# EUROPEAN PATENT SPECIFICATION

(11) **EP 4 126 173 B1**
(45) Date of publication and mention of the grant of the patent: **17.07.2024**
(21) Application number: 21720627.5
(22) Date of filing: 31.03.2021
(51) Int. Cl.: A61M 25/06, A61M 25/00

(54) **LOW TEMPERATURE HYDROPHILIC ADHESIVE FOR USE IN EXPANDABLE SHEATH FOR INTRODUCING AN ENDOVASCULAR DELIVERY DEVICE INTO A BODY**
BEI NIEDRIGER TEMPERATUR HYDROPHILER KLEBSTOFF ZUR VERWENDUNG IN EINER EXPANDIERBAREN HÜLLE ZUM EINFÜHREN EINER ENDOVASKULÄREN ABGABEVORRICHTUNG IN EINEN KÖRPER
ADHÉSIF HYDROPHILE À BASSE TEMPÉRATURE DESTINÉ À ÊTRE UTILISÉ DANS UNE GAINE EXPANSIBLE POUR INTRODUIRE UN DISPOSITIF D'ADMINISTRATION ENDOVASCULAIRE DANS UN CORPS

(30) Priority: 02.04.2020 US 202063004386 P
(43) Date of publication of application: 08.02.2023
(73) Proprietor: Edwards Lifesciences Corporation, Irvine, CA 92614-5688 (US)
(72) Inventor: LEE, Jeong, Soo, Irvine, CA 92614 (US); TAMIR, Ilan, Irvine, CA 92614 (US); GOLDBERG, Eran, 30889 Caesarea (IL)
(74) Representative: Eisenführ Speiser
(86) International application number: PCT/US2021/025038
(87) International publication number: WO 2021/202631

(56) References cited:
- US-A1- 2010 094 392
- US-A1- 2012 083 877
- US-A1- 2014 236 123

## Description

### CROSS-REFERENCE TO RELATED APPLICATIONS

This application claims the benefit of U.S. Provisional Application No. 63/004386, filed April 2, 2020.

### FIELD

The present application concerns aspects of a sheath for use with catheter-based technologies for repairing and/or replacing heart valves and delivering a prosthetic device, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (*e.g.*, the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath, and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes and the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

US 2012/0083877 A1 (US'877) discloses an expandable sheath for use in conjunction with a catheter assembly to introduce a prosthetic device, such as a heart valve, into a patient. The expandable sheath of US'877 has inner and outer layers, where a folded portion of the inner layer extends through a slit in the outer layer and a portion of the outer layer overlaps the folded portion of the inner layer. The expandable sheath of US'877 can include an elastic outer cover positioned outside the outer layer.

However, delivery and/or removal of prosthetic devices and other material to or from a patient still poses a significant risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

Accordingly, there remains a need in the art for an improved introducer sheath for endovascular systems used for implanting valves and other prosthetic devices.

### SUMMARY

The claimed invention is defined in independent claim 1 and relates to a sheath for delivering a medical device. Preferred configurations of the claimed sheath are defined in dependent claims 2 to 14. The claimed invention also relates to a method for making a sheath as defined in independent claim 15. Also disclosed herein are related aspects, examples, embodiments and arrangements useful for understanding the claimed invention.

The disclosed aspects are directed to an expandable sheath that can minimize trauma to the vessel by allowing for temporary expansion of a portion of the introducer sheath to accommodate a delivery system, followed by a return to the original diameter once the delivery system passes through. In some aspects, disclosed is a sheath having a smaller profile than that of prior art introducer sheaths. Furthermore, in certain aspects, the use of the disclosed sheaths can reduce the length of time a procedure takes and reduce the risk of a longitudinal or radial vessel tear or plaque dislodgement because only one sheath is required rather than several different sizes of sheaths. In some aspects, the expandable sheath can require only a single vessel insertion instead of requiring multiple insertions for the vessel's dilation.

Disclosed herein are aspects directed to a sheath for delivering a medical device. In some aspects, the sheath can have a proximal and a distal end and comprise: an expandable inner layer having an inner surface and an outer surface, wherein the inner surface of the expandable inner layer defines a lumen having a longitudinal axis and comprising at least one folded portion having an inner portion and outer portion; an outer layer having an inner surface and an outer surface and extending at least partially around the inner layer such that at least a first portion of the outer surface of the outer layer is positioned adjacent to the inner portion of the at least one folded portion of the inner layer, while a first portion of the inner surface of the outer layer is positioned adjacent to the outer portion of the at least one folded portion of the inner layer; a first polymer disposed between at least a portion of the inner layer and at least a portion of the outer layer, forming an intermediate layer, wherein, the first polymer exhibits a melting temperature from about 30 °C to about 45 °C; and wherein the at least one folded portion is configured to at least partially unfold during application of a radial outward force by passage of a medical device through the lumen of the inner layer.

In other aspects, the first polymer can be disposed between at least the first portion of the outer surface of the outer layer and the inner portion of the at least one folded portion of the inner layer. In yet another aspect, the first polymer can be disposed between at least the first portion of the inner surface of the outer layer and the outer portion of the at least one folded portion of the inner layer.

In still further aspects, the sheath can further comprise an outer jacket comprising an inner surface and outer surface extending at least partially around the outer layer such that the inner surface of the outer jacket overlies the outer surface of the outer layer.

Also disclosed herein are aspects comprising methods of making a sheath for delivering a medical device. In certain aspects, the methods disclosed herein are directed to making any of the disclosed herein sheaths.

In certain aspects, a method of making a sheath comprises providing a sheath comprising: an expandable inner layer having an inner surface and an outer surface, wherein the inner surface of the expandable inner layer defines a lumen having a longitudinal axis and comprising at least one folded portion having an inner portion and outer portion; and an outer layer having an inner surface and an outer surface and extending at least partially around the inner layer such that at least a first portion of the outer surface of the outer layer is positioned adjacent to the inner portion of the at least one folded portion of the inner layer, while a first portion of the inner surface of the outer layer is positioned adjacent to the outer portion of the at least one folded portion of the inner layer; applying a first polymer between at least a portion of the inner layer and at least a portion of the outer layer, thereby forming an intermediate layer, wherein, the first polymer exhibits a melting temperature from about 30 °C to about 45 °C and; wherein the at least one folded portion is configured to at least partially unfold during application of a radial outward force by passage of a medical device through the lumen of the inner layer.

In yet further aspects, the first polymer can be applied between the at least the first portion of the outer surface of the outer layer and the inner portion of the at least one folded portion of the inner layer. In still further aspects, the first polymer can be applied between the at least the first portion of the inner surface of the outer layer and the outer portion of the at least one folded portion of the inner layer. In still further aspect, the disclosed method further comprises positioning an outer jacket comprising an inner surface and outer surface such that it extends at least partially around the outer layer and such that the inner surface of the outer jacket overlies the outer surface of the outer layer.

The foregoing and other features and advantages of the disclosure will become more apparent from the following detailed description, which proceeds with reference to the accompanying figures.

### BRIEF DESCRIPTION OF THE DRAWINGS

**FIGURE 1** is an elevation view of a sheath according to the present disclosure along with an endovascular delivery apparatus for implanting a prosthetic valve.
**FIGURES 2A****, B,** and **D** are section views of a sheath for introducing a prosthetic device into a patient, and **FIGURE 2C** is a perspective view of one component of such a sheath, in one aspect.
**FIGURE 3** is an elevation view of the sheath shown in **FIGURE 2****.**
**FIGURES 4A-4B** are elevation views of a sheath according to the present disclosure, having varying outer diameters in alternative aspects.
**FIGURE 5** illustrates an elevation view of a sheath, expanded at a first location to accommodate a delivery system in one aspect.
**FIGURE 6** shows an elevation view of the sheath of claim 5, expanded at a second location farther down the sheath.
**FIGURE 7** shows a section view of a sheath that further comprises an outer covering or shell in another aspect.
**FIGURE 8** illustrates an elevation view of a sheath with an outer covering or shell in another aspect.
**FIGURE 9** illustrates a partial elevation view of an intermediate tubular layer that can be used to construct a sheath according to the present disclosure in one aspect.
**FIGURE 10** illustrates a partial elevation view of an intermediate tubular layer having a variable diamond design in another aspect.
**FIGURE 11** illustrates a partial elevation view of an intermediate tubular layer having a diamond design with spring struts in another aspect.
**FIGURE 12** illustrates a partial elevation view of an intermediate tubular layer having a diamond design with straight struts in another aspect.
**FIGURE 13** illustrates a partial elevation view of an intermediate tubular layer having a saw tooth design with spring struts in another aspect.
**FIGURE 14** illustrates a partial elevation view of an intermediate tubular layer having a saw tooth design with straight struts in another aspect.
**FIGURE 15** illustrates a partial elevation view of an intermediate tubular layer having a diamond design with straight struts in another aspect.
**FIGURE 16** illustrates a partial elevation view of an intermediate tubular layer having a helical or spiral design in another aspect.
**FIGURE 17** illustrates a partial elevation view of an intermediate tubular layer having a diamond design with non-straight struts in another aspect.
**FIGURE 18** illustrates a partial elevation view of an intermediate tubular layer having an alternative diamond design with non-straight struts in another aspect.
**FIGURE 19** illustrates a partial elevation view of an intermediate tubular layer having yet another diamond design with non-straight struts in another aspect.
**FIGURE 20** illustrates a partial elevation view of an intermediate tubular layer having a diamond design with struts in another aspect.
**FIGURE 21** illustrates a partial elevation view of an intermediate tubular layer having a design similar to that shown in **FIGURE 20**, but with additional struts in another aspect.
**FIGURE 22** illustrates a partial elevation view of an intermediate tubular layer having a diamond design with spiral struts in another aspect.
**FIGURE 23** illustrates a partial elevation view of an intermediate tubular layer having a diamond design with adjacent struts in another aspect.
**FIGURE 24** illustrates a section view of a sheath having a longitudinal notch in another aspect.
**FIGURE 25** shows a section view of a sheath having a longitudinal cut in the inner layer in another aspect.
**FIGURE 26** shows a perspective view of a sheath having a plurality of notches or cuts in the outer tubular layer in another aspect.
**FIGURE 27A** illustrates a section view of a sheath, wherein the outer tubular layer contains a longitudinal cut, and the inner layer extends into the gap created by the cut in the outer tubular layer, in an unexpanded configuration; and **FIGURES 27B-27E** show section views of various aspects of a sheath in the unexpanded configuration in another aspect.
**FIGURE 28** shows a section view of the sheath of **FIGURE 27A** in an expanded configuration.
**FIGURES 29A-29D** show section views of a sheath having overlapping sections in other aspects.
**FIGURE 30** illustrates a block diagram of a method of making a sheath according to the present disclosure in one aspect.
**FIGURE 31** illustrates a block diagram of a method of making a sheath according to the present disclosure in another aspect.
**FIGURES 32A-32H** illustrates section or elevation views of various method steps of the methods shown in **FIGURES 30-31****.**
**FIGURE 33** illustrates a plan view of a sheath having a partial slit or scoreline in another aspect.
**FIGURE 34** illustrates a plan view of a sheath having a partial slit or scoreline in another aspect.
**FIGURE 35** is an elevation view of an expandable sheath according to the present disclosure and representative housing.
**FIGURE 36** is an enlarged cutaway view of the distal end of the sheath of **FIGURE 35****.**
**FIGURES 37A-37B** is a section view of the distal end of the sheath of **FIGURE 35****,** taken along line 37-37 in **FIGURE 36****;** **FIGURE 37A** is a section view of the distal end of the sheath without an outer jacket, and **FIGURE 37B** is a section view of the distal end of the sheath with an outer jacket present.
**FIGURES 38A-38B** is a section view of a proximal section of the sheath of **FIGURE 35****,** taken along line 38-38 in **FIGURE 35****;** **FIGURE 38A** is a section view of the proximal end of the sheath without an outer jacket, and **FIGURE 38B** is a section view of the proximal end of the sheath with an outer jacket present.
**FIGURES 39A-39G** is a section view of the sheath of **FIGURE 35** in a rest (unexpanded) configuration, taken along line 39-39 in **FIGURE 35****;** **FIGURE 39A-**depicts a section view in one aspect without an exemplary first polymer, **FIGURES 39B-39G** depict a section view in various aspects with the presence of an exemplary first polymer.
**FIGURE 40A-40C** is a section view of the sheath of FIGURES 39A-C, in an expanded configuration; **FIGURE 40A** is a section view of the sheath of **FIGURE 39A** without an exemplary first polymer, **FIGURE 40B** is a section view of the sheath of **FIGURE 39B** with an exemplary first polymer present, **FIGURE 40C** is a section view of the sheath of **FIGURE 39E** with an exemplary first polymer present.

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting. In the following, 1 inch can be converted into 25.4 mm, and 1 lbs is equal to 0.4535924 kg.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "polymer" includes aspects having two or more such polymers unless the context clearly indicates otherwise.

It is also to be understood that the terminology used herein is for the purpose of describing particular aspects only and is not intended to be limiting. As used in the specification and in the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

References in the specification and concluding claims to parts by weight of a particular element or component in a composition or article, denotes the weight relationship between the element or component and any other elements or components in the composition or article for which a part by weight is expressed. Thus, in a composition or a selected portion of a composition containing 2 parts by weight of component X and 5 parts by weight component Y, X and Y are present at a weight ratio of 2:5, and are present in such ratio regardless of whether additional components are contained in the composition.

A weight percent of a component, unless specifically stated to the contrary, is based on the total weight of the formulation or composition in which the component is included.

Ranges can be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It should be further understood that the endpoints of each of the ranges are significant both in relation to the other endpoint, and independently of the other endpoint.

As used herein, the term "substantially," when used in reference to a composition, refers to at least about 80%, at least about 85%, at least about 90%, at least about 91 %, at least about 92%, at least about 93%, at least about 94%, at least about 95%, at least about 96%, at least about 97%, at least about 98%, at least about 99%, or about 100% by weight, based on the total weight of the composition, of a specified feature or component.

As used herein, the term "substantially," in, for example, the context "substantially free" refers to a composition having less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the terms "substantially identical reference composition" or "substantially identical reference article" refer to a reference composition or article comprising substantially identical components in the absence of an inventive component. In another exemplary aspect, the term "substantially," in, for example, the context "substantially identical reference composition," refers to a reference composition comprising substantially identical components and wherein an inventive component is substituted with a common in the art component. For example, a substantially identical reference sheath can comprise a sheath comprising substantially identical components but without the presence of the first polymer. In a still further example, a substantially identical reference sheath can comprise a sheath comprising substantially identical components but without the presence of the first polymer and the second polymer; or a sheath comprising substantially identical components but without the presence of the first polymer, the second polymer and/or the third polymer.

Further, the terms "coupled" and "associated" generally mean electrically, electromagnetically, and/or physically (e.g., mechanically or chemically) coupled or linked and do not exclude the presence of intermediate elements between the coupled or associated items.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### SHEATH

In some aspects, the disclosure is directed to an expandable sheath that can minimize trauma to the vessel by allowing for temporary expansion of a portion of the introducer sheath to accommodate the delivery system, followed by a return to the original diameter once the device passes through. In certain aspects and as disclosed herein, a sheath has a smaller profile (e.g., a smaller diameter in the rest configuration) than that of prior art introducer sheaths. Furthermore, the sheaths disclosed in certain aspects of the present disclosure can reduce the length of time a procedure takes and reduce the risk of a longitudinal or radial vessel tear or plaque dislodgement because only one sheath is required rather than several different sizes of sheaths. In yet other aspects, the use of the disclosed expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices (e.g., stents, prosthetic heart valves, stented grafts, etc.) into many types of vascular and non-vascular body lumens (e.g., veins, arteries, esophagus, ducts of the biliary tree, intestine, urethra, fallopian tube, other endocrine or exocrine ducts, etc.).

In some aspects, the sheath disclosed herein for introducing a prosthetic device can comprise an inner layer and an outer layer. In certain aspects, at least a portion of the sheath can be designed or configured to locally expand from a first diameter to a second diameter as the prosthetic device is pushed through a lumen of the sheath and then at least partially return to the first diameter once the prosthetic device has passed through. In certain aspects, the sheath can additionally comprise an elastic outer cover disposed about the outer layer. Yet, in still other aspects, the sheath can include an outer jacket disposed about the outer layer.

One aspect, the sheath for introducing a prosthetic device into a body, can comprise a continuous inner layer defining a lumen therethrough, the inner layer having a folded portion and a discontinuous outer layer having an overlapping portion and an underlying portion. In some aspects, the inner layer can have at least two folded portions. The outer layer can be configured such that the overlapping portion overlaps the underlying portion, wherein at least a portion of the folded portion of the inner tubular layer is positioned between the overlapping and underlying portions. In yet other aspects, at least a portion of the sheath can be configured to expand to accommodate the prosthetic device.

In some aspects, at least a portion of the sheath can be configured such that a plurality of segments of the sheath each can locally expand one at a time from a rest configuration having a first diameter to an expanded configuration having a second diameter that is larger than the first diameter, to facilitate passage of the prosthetic device through the lumen of the inner layer. In certain aspects, each segment can have a length defined along the longitudinal axis of the sheath, and each segment of the sheath can be configured to at least partially return to the first diameter once the prosthetic device has passed through.

In some aspects, when each segment of the sheath is in the expanded configuration, a length of the folded portion corresponding to the length of the segment at least partially unfolds (e.g., by separating and/or straightening). In still further aspects, a length of the overlapping portion corresponding to the length of the segment can be configured to move with respect to the underlying portion when each segment of the sheath expands from the rest configuration to the expanded configuration.

In some exemplary aspects and as disclosed in detail below, the sheath can further comprise an elastic outer cover disposed on an external surface of the outer layer. In still further aspects, the elastic outer cover can be the outer jacket of the sheath. The elastic outer cover can comprise, for example, heat shrink tubing. In certain aspects, and as disclosed in detail below, the sheath can also comprise one or more radiopaque markers or fillers, such as a C-shaped band positioned between the inner and outer layers near the distal end of the sheath. In certain exemplary and unlimiting aspects, a soft tip can be secured to the distal end of the sheath.

In some aspects and as disclosed below, the inner layer can include at least one folded portion and at least one weakened portion. In certain aspects, the sheath can comprise a discontinuous outer layer. In such exemplary aspects, the discontinuous outer layer can have an outer surface, and an inner surface and a longitudinal gap, and a portion of the inner layer can extend through the longitudinal gap. The at least one folded portion of the inner layer can be positioned adjacent to a portion of the outer surface of the outer layer. In some aspects, the weakened portion can comprise a scoreline along at least a portion of the inner layer and/or a slit along at least a portion of the inner layer. The weakened portion can be positioned at the at least one folded portion of the inner layer. In some aspects, the longitudinal gap can be positioned between a first end and a second end of the outer layer.

In some exemplary aspects and as also disclosed in detail below, an expandable sheath can comprise a hydrophilic inner liner defining a generally horseshoe-shaped lumen therethrough, the inner liner, including at least two weakened portions and an elastic cover, positioned radially outward of the inner liner. In some aspects, when the sheath is in the expanded configuration, the inner liner splits apart at the weakened portions so as to form a discontinuous inner liner.

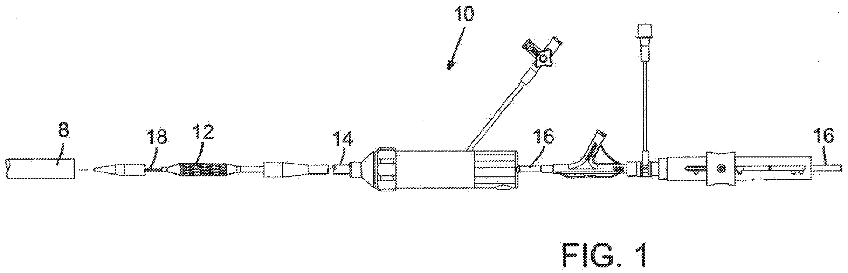

**FIG. 1** illustrates an exemplary sheath **8** according to the present disclosure, in use with a representative delivery apparatus **10**, for delivering a prosthetic device **12**, such as a tissue heart valve to a patient. The apparatus **10** can include a steerable guide catheter **14** (also referred to as a flex catheter), a balloon catheter **16** extending through the guide catheter **14**, and a nose catheter **18** extending through the balloon catheter **16.** The guide catheter **14**, the balloon catheter **16**, and the nose catheter **18** in the illustrated aspect are adapted to slide longitudinally relative to each other to facilitate delivery and positioning of the valve **12** at an implantation site in a patient's body, as described in detail below.

In certain aspects, the sheath disclosed herein can comprise a proximal end and a distal end opposite one another. Generally, in certain aspects, sheath **8** can be inserted into a vessel, such as the transfemoral vessel, pass through the skin of the patient, such that the distal end of the sheath **8** can be inserted into the vessel. Sheath **8** can also comprise a hemostasis valve at/or near the opposite, proximal end of the sheath. Yet, in further aspects, the delivery apparatus **10** can be inserted into the sheath **8**, and the prosthetic device **12** can then be delivered and implanted within the patient.

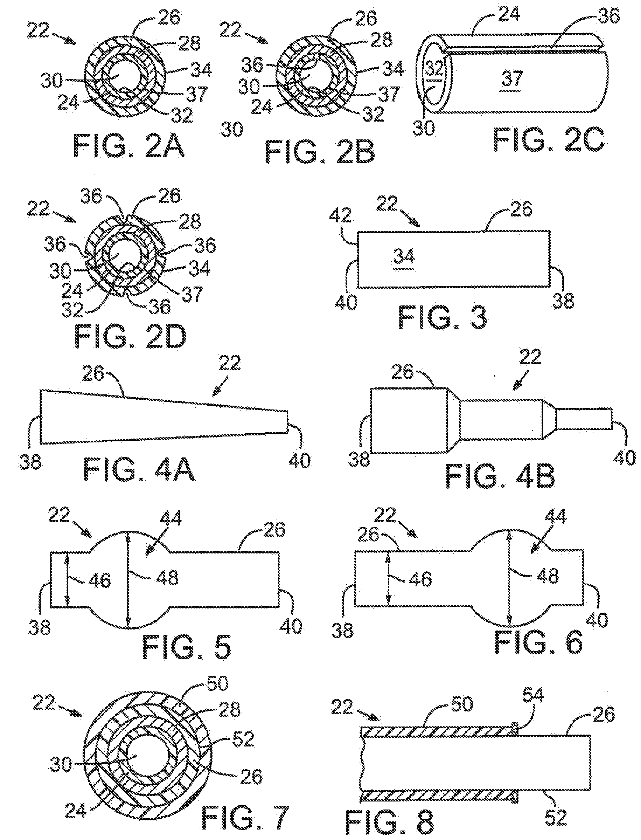

**FIGS. 2A, 2B,** and **2D** show exemplary section views of a sheath 22, in one aspect, for use with a delivery apparatus such as that shown in **FIG. 1****.** **FIG. 2C** shows a perspective view of one aspect of an inner layer **24** for use with the sheath **22** in one aspect. Sheath **22** includes an inner layer, such as inner polymeric tubular layer **24,** an outer layer, such as outer polymeric tubular layer **26**, and an intermediate tubular layer **28** disposed between the inner and outer polymeric tubular layers **24, 26.** The sheath **22** defines a lumen **30** through which a delivery apparatus can travel into a patient's vessel in order to deliver, remove, repair, and/or replace a prosthetic device. Such introducer sheaths **22** can also be useful for other types of minimally invasive surgery, such as any surgery requiring introduction of an apparatus into a subject's vessel. For example, and without limitations, the sheath **22** also can be used to introduce other types of delivery apparatus for placing various types of intraluminal devices (*e.g.*, stents, stented grafts, etc.) into many types of vascular and non-vascular body lumens (*e.g.*, veins, arteries, esophagus, ducts of the biliary tree, intestine, urethra, fallopian tube, other endocrine or exocrine ducts, etc.).

In certain aspects, the inner layer can comprise polytetrafluoroethylene (PTFE), polyimide, polyetheretherketone (PEEK), polyurethane, nylon, polyethylene, polyamide, or combinations thereof.

In other aspects, the outer layer can comprise PTFE, polyimide, PEEK, polyurethane, nylon, polyethylene, polyamide, polyether block amides, polyether block ester copolymer, thermoset silicone, latex, poly-isoprene rubbers, high-density polyethylene (HDPE), Tecoflex, or combinations thereof.

In certain exemplary and unlimiting aspects, the outer polymeric tubular layer **26** and/or the inner polymeric tubular layer **24** can comprise, for example, PTFE (e.g., Teflon^{®}), polyimide, PEEK, polyurethane, nylon, polyethylene, polyamide, polyether block amides (e.g., PEBAX^{®}), polyether block ester copolymer, polyesters, fluoropolymers, polyvinyl chloride, thermoset silicone, latex, poly-isoprene rubbers, polyolefin, other medical grade polymers, or combinations thereof.

In some exemplary aspects, the inner layer can comprise PTFE and the outer layer can comprise a combination of HDPE and/or Tecoflex. In yet other exemplary aspects, the inner and outer layers can be thermally fused together. In some exemplary and unlimiting aspects, the inner layer can comprise a woven fabric and/or braided filaments such as yarn filaments of PTFE, PET, PEEK, and/or nylon.

The intermediate tubular layer **28** can comprise a shape memory alloy such as Nitinol and/or stainless steel, cobalt, chromium, spectra fiber, polyethylene fiber, aramid fiber, or combinations thereof.

In certain aspects, the inner polymeric tubular layer **24** can advantageously be provided with a low coefficient of friction on its inner surface. For example, the inner polymeric tubular layer **24** can have a coefficient of friction of less than about 0.5, less than about 0.4, less than about 0.3, less than about 0.2, less than about 0.1, less than about 0.05, or even less than about 0.01. In some aspects, a sheath **22** can include a lubricious liner on the inner surface **32** of the inner polymeric tubular layer **24.** Such a liner can facilitate passage of a delivery apparatus through the lumen **30** of the sheath **22.** Examples of suitable lubricious liners include materials that can reduce the coefficient of friction of the inner polymeric tubular layer **24**, such as PTFE, polyethylene, polyvinylidene fluoride, and combinations thereof. Suitable materials for a lubricious liner also include other materials desirably having a coefficient of friction of about 0.5 or less, about 0.4 or less, about 0.3 or less, about 0.2 or less, about 0.1 or less, of about 0.09 or less, about 0.08 or less, about 0.07 or less, about 0.05 or less, about 0.04 or less, about 0.03 or less, about 0.02 or less, or about 0.01 or less.

The inner diameter of the intermediate tubular layer **28** can vary depending on the application and size of the delivery apparatus and prosthetic device. In some aspects, the inner diameter ranges from about 0.005 inches to about 0.400 inches, including exemplary values of about 0.01 about 0.02, about 0.03, about 0.04, about 0.05, about 0.06, about 0.07, about 0.08, about 0.09, about 0.1, about 0.2, and about 0.3 inches. The thickness of the intermediate tubular layer **28** can also be varied depending on the desired amount of radial expansion, as well as the strength required. For example, and without limitations, the thickness of the intermediate tubular layer **28** can be from about 0.002 inches to about 0.025 inches, including exemplary values of about 0.003, about 0.004, about 0.005, about 0.006, about 0.007, about 0.008, about 0.009, about 0.01, about 0.011, about 0.012, about 0.013, about 0.014, about 0.015, about 0.016, about 0.017, about 0.018, about 0.019, about 0.020, about 0.021, about 0.022, about 0.023, and about 0.024 inches.

The thicknesses of the inner polymeric tubular layer **24** and the outer polymeric tubular layer **26** can also be varied depending on the particular application of the sheath **22.** In some aspects, the thickness of the inner polymeric tubular layer 24 ranges from about 0.0005 inches to about 0.010 inches, including exemplary values of about 0.0006, about 0.0007, about 0.0008, about 0.0009, about 0.001, about 0.002, about 0.003, about 0.004, about 0.005, about 0.006, about 0.007, about 0.008, about 0.009 inches, and in one particular aspect, the thickness can be about 0.002 inches. Outer polymeric tubular layers **26** can have a thickness of from about 0.002 inches to about 0.015 inches, including exemplary values of about 0.003, about 0.004, about 0.005, about 0.006, about 0.007, about 0.008, about 0.009, about 0.01 inches, and in one particular aspect, the outer polymeric tubular layer **26** has a thickness of about 0.010 inches.

The hardness of each layer of the sheath **22** can also be varied depending on the particular application and desired properties of the sheath **22.** In some aspects, the outer polymeric tubular layer **26** has a Shore hardness of from about 25 Durometer to about 75 Durometer, including exemplary values of about 30 Durometer, about 35 Durometer, about 40 Durometer, about 45 Durometer, about 50 Durometer, about 55 Durometer, about 60 Durometer, about 65 Durometer, and about 70 Durometer.

Additionally, in some aspects, the sheath **22** can comprise, as mentioned above, an exterior hydrophilic coating on the outer surface **34** of the outer polymeric tubular layer **26.** Such a hydrophilic coating can facilitate insertion of the sheath **22** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings, are also suitable for use with the sheath **22.**

In some aspects, the outer surface **34** of the outer polymeric tubular layer **26** can be modified. For example, surface modifications such as plasma etching can be performed on the outer surface **34.** It is understood, however, that plasma etching is only an exemplary surface modification method, and other methods such as laser ablation, chemical etching, etc., can also be employed. Similarly, in certain aspects, if desired, other surfaces, both outer and inner, can be surface modified. In some aspects, surface modification can improve adhesion between the layers in the areas of the modification.

The sheath **22** also can have at least one radiopaque filler or marker. The radiopaque filler or marker can be associated with the outer surface **3**4 of the outer polymeric tubular layer **26.** Alternatively, the radiopaque filler or marker can be embedded or blended within the outer polymeric tubular layer **24.** Similarly, the radiopaque filler or marker can be associated with a surface of the inner polymeric tubular layer **24** or the intermediate tubular layer **28** or embedded within either or both of those layers.

In some aspects, suitable materials for use as a radiopaque filler or marker include, for example, barium sulfite, bismuth trioxide, titanium dioxide, bismuth subcarbonate, or combinations thereof. In certain aspects, the radiopaque filler can be mixed with or embedded in the material used to form the outer polymeric tubular layer **26**, and can comprise from about 5% to about 45% by weight of the outer polymeric tubular layer, including exemplary values of about 10%, about 15%, about 20%, about 25%, about 30%, about 35%, and about 40% by weight of the outer polymeric tubular layer. The more or less radiopaque material can be used in some aspects, depending on the particular application.

In some aspects, the inner polymeric tubular layer **24** can comprise a substantially uniform cylindrical tube. In alternative aspects, the inner polymeric tubular layer **24** can have at least one section of discontinuity along its longitudinal axis to facilitate radial expansion of the inner polymeric tubular layer **24.** For example, the inner polymeric tubular layer **24** can be provided with one or more longitudinal notches and/or cuts **36** extending along at least a portion of the length of the sheath **22.** Such notches or cuts **36** can facilitate radial expansion of the inner polymeric tubular layer **24**, thus accommodating passage of a delivery apparatus or other device. Such notches and/or cuts **36** can be provided near the inner surface **32**, near the outer surface **37**, and/or substantially through the entire thickness of the inner polymeric layer **24.** In aspects with a plurality of notches and/or cuts **36**, such notches and/or cuts **36** can be positioned such that they are substantially equally spaced from one another circumferentially around the inner polymeric layer **24.** Alternatively, notches and cuts **36** can be spaced randomly in relation to one another or in any other desired pattern. Some or all of any provided notches and/or cuts **36** can extend longitudinally along substantially the entire length of the sheath **22.** Alternatively, some or all of any provided notches and/or cuts **36** can extend longitudinally only along a portion of the length of the sheath **22.**

As shown in **FIGS. 2B** and **2C** (which illustrates only the inner polymeric tubular layer **24**), in some aspects, the inner polymeric tubular layer **24** contains at least one notch or cut **36** that extends longitudinally and parallel to an axis defined by the lumen **30**, extending substantially the entire length of the sheath **22.** Thus, upon introduction of a delivery apparatus, the inner polymeric tubular layer **24** can split open along the notch and/or cut **36** and expand, thus accommodating the delivery apparatus.

Additionally or alternatively, in some aspects, and as shown in **FIG. 2D**, the outer polymeric tubular layer **26** can comprise one or more notches and/or cuts **36.** Notches and/or cuts **36**, in some aspects, do not extend through the entire thickness of the outer tubular layer **26.** The notches and/or cuts **36** can be separable upon radial expansion of the sheath **22.** The outer polymeric tubular layer **26** can be retractable longitudinally or able to be pulled back away from the intermediate tubular layer **28** and the inner polymeric tubular layer **24.** In aspects with a retractable outer polymeric tubular layer **26**, the outer polymeric tubular layer **26** can be retracted to accommodate or facilitate passage of a delivery apparatus through the lumen **30**, and then can be replaced to its original position on the sheath **22.**

**FIG. 3** illustrates an exemplary elevation view of the sheath **22** shown in **FIG. 2A****.** In this view, only the outer polymeric tubular layer **26** is visible. The sheath **22** comprises a proximal end **38** and a distal end **40** opposite the proximal end **38.** The sheath **22** can include a hemostasis valve inside the lumen of the sheath **22**, at or near the proximal end **38** of the sheath **22.** Additionally, the sheath **22** can comprise a soft tip **42** at the distal end **40** of the sheath **22.** Such a soft tip **42** can be provided with a lower hardness than the other portions of the sheath **22.** In some aspects, the soft tip **42** can have a Shore hardness from about 25 D to about 40 D, including exemplary values of about 26 D, about 27 D, about 28 D, about 29 D, about 30 D, about 31 D, about 32 D, about 33 D, about 34 D, about 35 D, about 36 D, about 37 D, about 38 D, and about 39 D.

In some aspects, the unexpanded original outer diameter can be substantially constant along at least a majority of the length of the sheath. While in other aspects, the unexpanded original outer diameter of the sheath can decrease along a gradient from the proximal end to the distal end of the sheath.

For example, as shown in **FIG. 3**, the unexpanded original outer diameter of the sheath **22** can be substantially constant across the length of the sheath **22**, substantially from the proximal end **38** to the distal end **40.** In alternative aspects, such as the ones illustrated in **FIGS. 4A-4B**, the original unexpanded outer diameter of the sheath **22** can decrease from the proximal end **38** to the distal end **40**. As shown in the aspect in **FIG. 4A**, the original unexpanded outer diameter can decrease along a gradient, from the proximal end **38** to the distal end **40.** In alternative aspects, as shown in **FIG. 4B**, the original unexpanded outer diameter of sheath **22** can incrementally step down along the length of the sheath **22**, wherein the largest original unexpanded outer diameter is near the proximal end **38,** and the smallest original unexpanded outer diameter is near the distal end **40** of the sheath **22.**

As shown in **FIGS. 5-6**, the sheath **22** can be designed to locally expand as the prosthetic device is passed through the lumen of the sheath **22** and then substantially return to its original shape once the prosthetic device has passed through that portion of the sheath **22.** For example, and without limitations, **FIG. 5** illustrates a sheath **22** having a localized bulge **44,** representative of a device being passed through the internal lumen of the sheath **22.** **FIG. 5** shows the device close to the proximal end **38** of the sheath **22**, close to the area where the device is introduced into the sheath **22.** **FIG. 6** shows the sheath **22** of **FIG. 5**, with the device having progressed further along the sheath **22.** The localized bulge **44** is now closer to the distal end **40** of the sheath **22** and thus is about to be introduced to a patient's vessel. As evident from **FIGS. 5** and **6****,** once the localized bulge associated with the device has passed through a portion of the lumen of the sheath **22**, that portion of the sheath **22** can automatically return to its original shape and size, at least in part due to the materials and structure of the sheath **22.**

The sheath **22** has an unexpanded inner diameter equal to the inner diameter of the inner polymeric tubular layer (not visible in **FIGS. 5-6**) and an unexpanded outer diameter **46** equal to the outer diameter of the outer polymeric tubular layer **26.** The sheath **22** is designed to be expanded to an expanded inner diameter and an expanded outer diameter **48,** which are larger than the unexpanded inner diameter and the unexpanded outer diameter **46**, respectively. In one representative aspect, the unexpanded inner diameter is about 16 Fr, and the unexpanded outer diameter 46 is about 19 Fr, while the expanded inner diameter is about 26 Fr, and the expanded outer diameter 48 is about 29 Fr. Different sheaths **22** can be provided with different expanded and unexpanded inner and outer diameters, depending on the size requirements of the delivery apparatus for various applications. Additionally, some aspects can provide more or less expansion depending on the particular design parameters, the materials, and/or configurations used.

In some aspects, and as shown in section in **FIG. 7** and in elevation in **FIG. 8**, the sheath **22** can additionally comprise an outer covering, such as outer polymeric covering **50**, disposed on the outer surface **52** of the outer polymeric tubular layer **26.** In such exemplary and unlimiting aspects, the outer polymeric covering **50** can provide a protective covering for the underlying sheath **22.** In some aspects, the outer polymeric covering **50** can contain a self-expandable sheath in a crimped or constrained state and then release the self-expandable sheath upon removal of the outer polymeric covering **50.** For example, in some aspects of a self-expandable sheath, the intermediate layer **28** can comprise Nitinol and/or other shape memory alloys, and the intermediate layer **28** can be crimped or radially compressed to a reduced diameter within the outer polymeric tubular layer **26** and the outer polymeric covering **50.** Once the self-expandable sheath is at least partially inserted into a patient's vessel, the outer polymeric covering **50** can be slid back, peeled away, or otherwise at least partially removed from the sheath. To facilitate removal of the outer polymeric covering **50**, a portion of the outer polymeric covering **50** can remain outside the patient's vessel, and that portion can be pulled back or removed from the sheath to allow the sheath to expand. In some aspects, substantially the entire outer polymeric covering **50** can be inserted, along with the sheath, into a patient's vessel. In these aspects, an external mechanism attached to the outer polymeric covering **50** can be provided, such that the outer polymeric covering can be at least partially removed from the sheath once the sheath is inserted into a patient's vessel.

Once no longer constrained by the outer polymeric covering **50**, the radially compressed intermediate layer **28** can self-expand, causing expansion of the sheath along the length of the intermediate layer **28.** In some aspects, portions of the sheath can radially collapse, at least partially returning to the original crimped state, as the sheath is being withdrawn from the vessel after completion of the surgical procedure. In yet other aspects, such collapse can be facilitated and/or encouraged by an additional device or layer that, in some aspects, can be mounted onto a portion of the sheath prior to the sheath's insertion into the vessel.

The outer polymeric covering **50**, in some aspects, is not adhered to the other layers of the sheath **22.** For example, the outer polymeric covering **50** may be slidable with respect to the underlying sheath, such that it can be easily removed or retracted from its initial position on the sheath **22.**

As seen in **FIG. 8**, the outer polymeric covering **50** can include one or more peel tabs **54** to facilitate manual removal of the outer polymeric covering **50.** The outer polymeric covering **50** can be automatically or manually retractable and/or splittable to facilitate radial expansion of the sheath **22.** Peel tabs **54** can be located approximately **90** degrees from any cut or notch present in the outer polymeric covering **50** and approximately **180** degrees offset from one another. In alternative aspects, the peel tabs **54** can extend substantially around the circumference of the outer polymeric covering **50**, thus resulting in a single circular peel tab **54.**

Suitable materials for the outer polymeric covering **50** are similar to those materials suitable for the inner polymeric tubular layer and the outer polymeric tubular layer as described above. In still further aspects, the materials can include PTFE and/or high density polyethylene.

Turning now to the intermediate tubular layer **28**, several different configurations are possible. In some aspects, the intermediate tubular layer **28** can be a thin, hollow, substantially cylindrical tube comprising an arrangement, pattern, structure, or configuration of wires or struts, however other geometries can also be used. The intermediate tubular layer **28** can extend along substantially the entire length of the sheath **22**, or alternatively, can extend only along a portion of the length of sheath **22.** Suitable wires can be round, ranging from about 0.0005 inches thick to about 0.10 inches thick, including exemplary values of about 0.0006, about 0.0007, about 0.0008, about 0.0009, about 0.001, about 0.002, about 0.003, about 0.004, about 0.005, about 0.006, about 0.007, about 0.008, about 0.009, about 0.01, about 0.02, about 0.03, about 0.04, about 0.05, about 0.06, about 0.07, about 0.08, and about 0.09 inches, or flat, ranging from about 0.0005 inches x 0.003 inches to about 0.003 inches x 0.007 inches, including any exemplary values between the two foregoing values. However, other geometries and sizes are also suitable for certain aspects. If a braided wire is used, the braid density can be varied. Some aspects have a braid density of about thirty picks per inch to about eighty picks per inch and can include up to thirty-two wires in various braid patterns.

In one exemplary aspect, an intermediate tubular layer can comprise a braided Nitinol composite, which is at least partially encapsulated by an inner polymeric tubular member and an outer polymeric tubular member disposed on inner and outer surfaces of the intermediate tubular layer, respectively. Such encapsulation by polymeric layers can be accomplished by, for example, fusing the polymeric layers to the intermediate tubular layer or dip coating the intermediate tubular layer. In some aspects, an inner polymeric tubular member, an intermediate tubular layer, and an outer polymeric tubular layer can be arranged on a mandrel, and the layers can then be thermally fused or melted into one another by placing the assembly in an oven or otherwise heating it. The mandrel can then be removed from the resulting sheath. In yet other aspects, dip coating can be used to apply an inner polymeric tubular member to the surface of a mandrel. The intermediate tubular layer can then be applied, and the inner polymeric tubular member allowed to cure. The assembly can then be dip coated again, such as to apply a thin coating of, for example, polyurethane, which will become the outer polymeric tubular member of the sheath. The sheath can then be removed from the mandrel.

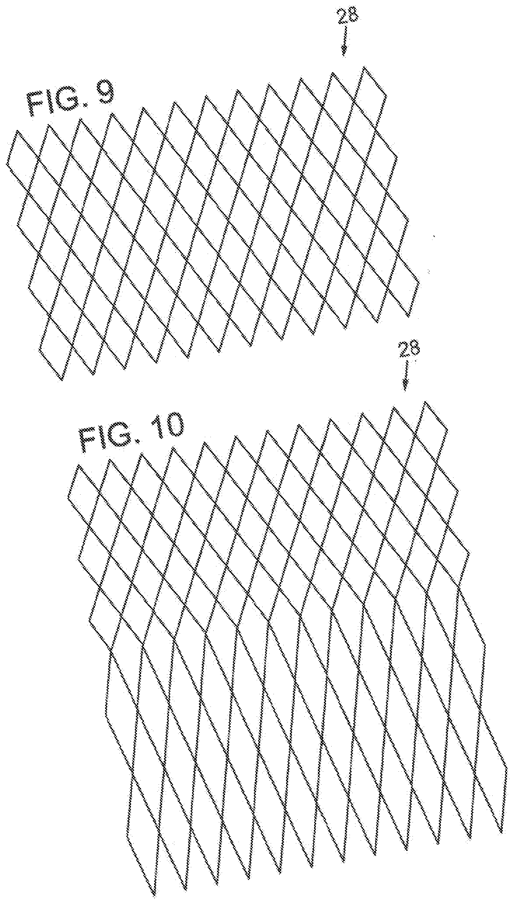

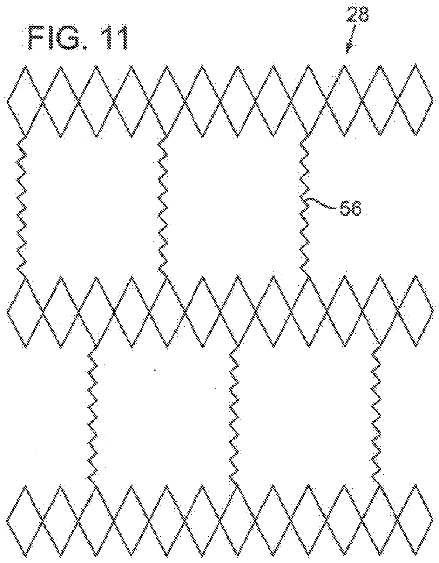

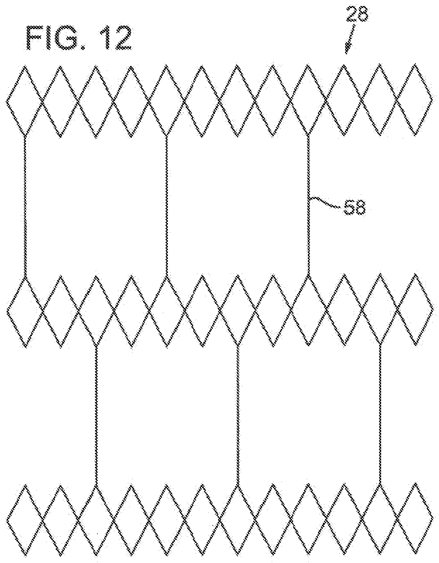

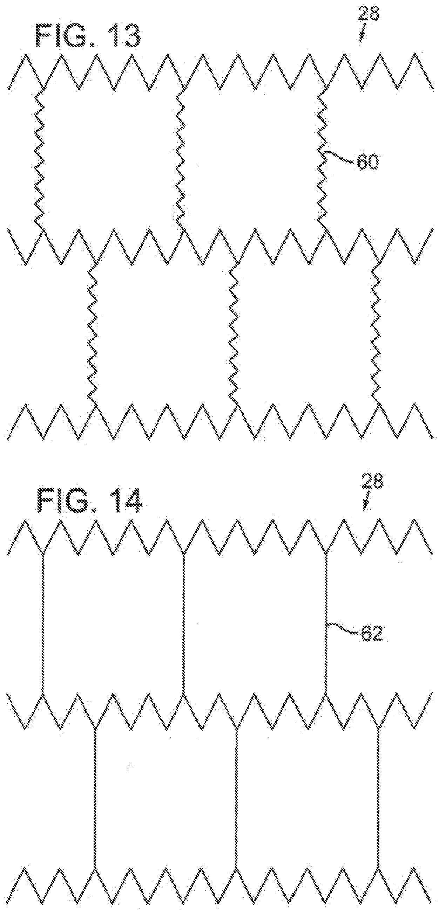

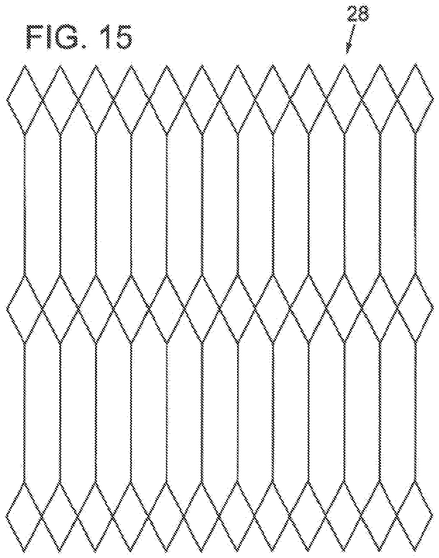

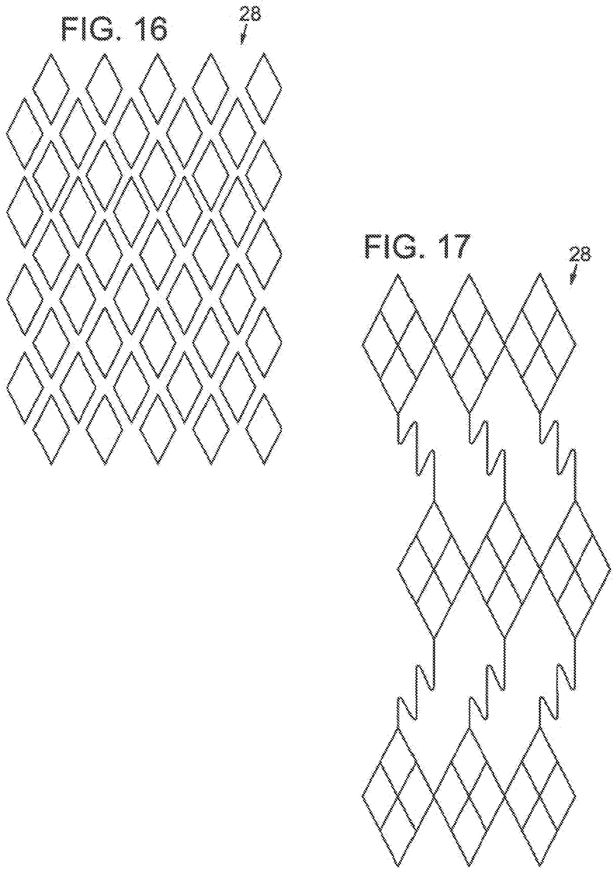

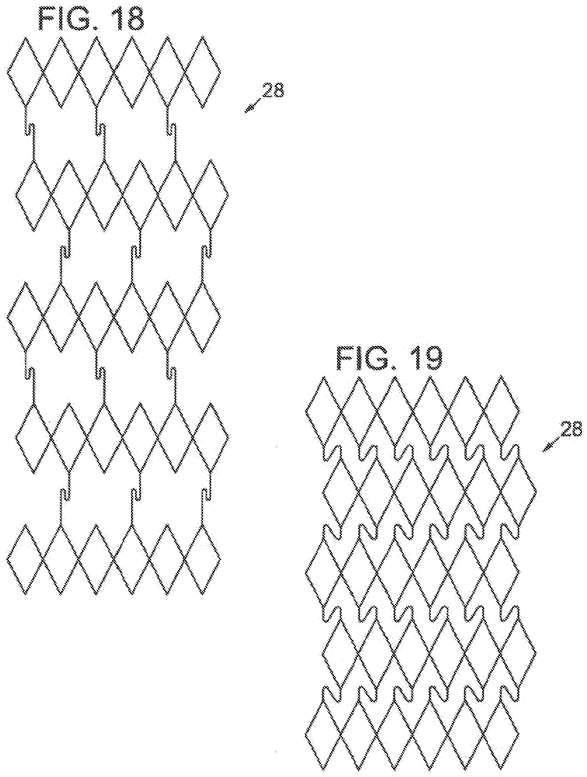

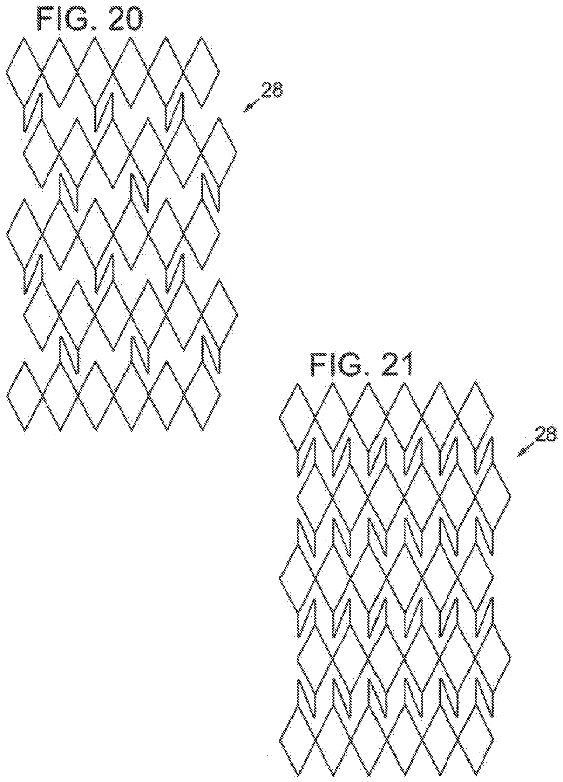

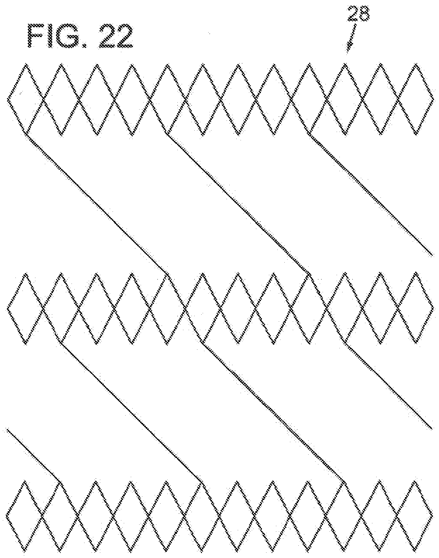

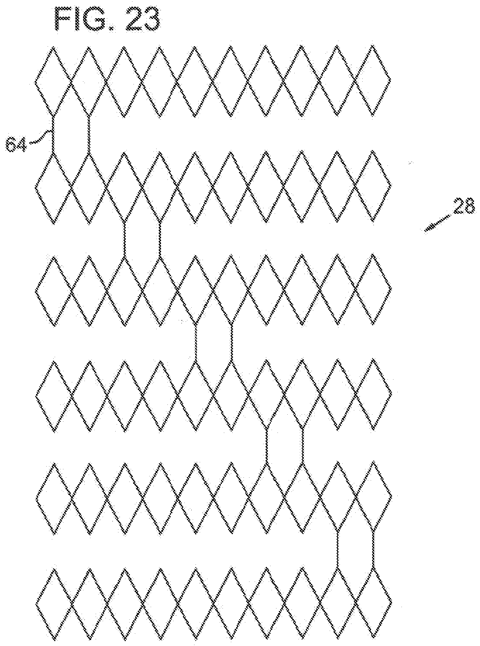

Additionally, in some aspects, the intermediate tubular layer **28** can be, for example, braided or laser cut to form a pattern or structure, such that the intermediate tubular layer **28** is amenable to radial expansion. **FIGS. 9-23** illustrate exemplary partial elevation views of various structures for the intermediate tubular layer. Some illustrated structures, such as those shown in **FIGS. 11-14** and **23** can include at least one discontinuity. For example, the struts **56**, **58**, **60**, **62**, **64,** shown in **FIGS. 11****,** **12****,** **13, 14****,** and **23**, respectively, can result in a discontinuous intermediate tubular layer **28** in that the struts **56, 58, 60, 62, 64** separate adjacent sections of the intermediate tubular layer **28** from each other, where the sections are spaced apart from each other along a longitudinal axis parallel to the lumen of the sheath. Thus, the structure of the intermediate tubular layer **28** can vary from section to section, changing along the length of the sheath.

The structures shown in **FIGS. 9-23** are not necessarily drawn to scale. Components and elements of the structures can be used alone or in combination within a single intermediate tubular layer **28.** The scope of the intermediate tubular layer **28** is not meant to be limited to these particular structures; they are merely exemplary aspects.

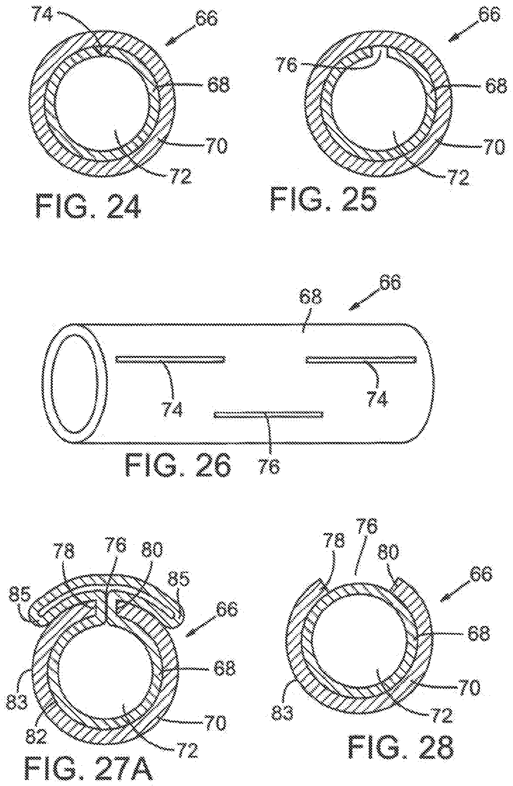

Alternative aspects of a sheath for introducing a prosthetic device are also described. For example, **FIGS. 24-26** illustrate a section view and a perspective view, respectively, of a sheath **66** for introducing a prosthetic device into a body. The sheath **66** can comprise an inner layer, such as inner polymeric layer **68**, an outer layer, such as polymeric tubular layer **70**, and a hemostasis valve (not shown). The inner polymeric layer **68** and the outer polymeric tubular layer **70** can at least partially enclose a lumen **72**, through which a delivery apparatus and prosthetic device can pass from outside the patient's body into the patient's vessel. Either or both of the inner polymeric layer **68** and the outer polymeric layer **70** can be provided with at least one longitudinal notch and/or cut to facilitate radial expansion of the sheath.

For example, **FIG. 24** illustrates a longitudinal notch **74** in the inner polymeric layer **68** that can facilitate radial expansion of the sheath **66.** The longitudinal notch **74** can separate or split open completely upon application of a radial force due to insertion of a delivery apparatus or prosthetic device. Similarly, **FIG. 25** illustrates a longitudinal cut **76** in the inner polymeric layer **68** that can also facilitate radial expansion of the sheath **66.** The outer polymeric layer **70** can, additionally or alternatively, comprise one or more longitudinal cuts **76** or notches **74.** Such cuts and/or notches, whether in the inner polymeric layer **68** or the outer polymeric layer **70**, can extend substantially through the entire thickness of the layer, or can extend only partially through the thickness of the layer. The cuts and/or notches can be positioned at or near the inner or outer surface, or both surfaces, of the inner and/or outer polymeric layers **68, 70.**

**FIG. 26** illustrates a perspective view of an inner polymeric layer **68,** in one aspect, with longitudinal notches **74** and a longitudinal cut **76.** More or fewer notches **74** and/or cuts **76** can be provided. For clarity, the outer polymeric layer **70** is not shown in **FIG. 26****.** As shown in **FIG. 26**, longitudinal notches **74** and/or cuts **76** can extend only along a portion of the length of sheath **66.** In alternative aspects, one or more notches **74** and/or cuts **76** can extend substantially along the entire length of the sheath **66.** Additionally, notches **74** and/or cuts **76** can be positioned randomly or patterned.

In one exemplary and unlimiting aspect, the sheath **66** can have a notch or cut in the outer polymeric layer **70** or the inner polymeric layer **68** that extends longitudinally along approximately 75% of the length of the sheath **66.** If such a notch or cut extends only partially through the associated layer, it can have a relatively low tear force, such as a tear force of about 0.5 lbs., so that the notch splits open relatively easily during use.

The inner polymeric layer **68** and the outer polymeric layer **70** can optionally be adhered together or otherwise physically associated with one another. The amount of adhesion between the inner polymeric layer **68** and the outer polymeric layer **70** can be variable over the surfaces of the layers. For example, little to no adhesion can be present at areas around or near any notches and/or cuts present in the layers so as not to hinder the radial expansion of the sheath **66.** Adhesion between the layers can be created by, for example, thermal bonding and/or coatings. In some aspects, the sheath **66** can be formed from an extruded tube, which can serve as the inner polymeric layer **68.** The inner polymeric layer **68** can be surface-treated, such as by plasma etching, chemical etching, laser ablation, or other suitable methods of surface treatment. By treating the surface of the inner polymeric layer **68**, the outer surface of the inner polymeric layer **68** can have areas with altered surface angles that can provide better adhesion between the inner polymeric layer **68** and the outer polymeric layer **70.** The treated inner polymeric layer can be dip coated in, for example, a polyurethane solution to form the outer polymeric layer **70.** In some configurations, the polyurethane may not adhere well to untreated surface areas of the inner polymeric layer **68.** Thus, by surface treating only surface areas of the inner polymeric layer **68** that are spaced away from the areas of expansion (*e.g.*, the portion of the inner polymeric layer **68** near notches **74** and/or cuts **76**), the outer polymeric layer **70** can be adhered to some areas of the inner polymeric layer **68**, while other areas of the inner polymeric layer **68** remain free to slide relative to the outer polymeric layer **70**, thus allowing for expansion of the diameter of the sheath **66.** Thus, areas around or near any notches **74** and/or cuts **76** can experience little to no adhesion between the layers, while other areas of the inner and outer polymeric layers **68, 70** can be adhesively secured or otherwise physically associated with each other.

In some aspects, the structures illustrated in **FIGS. 24-26** can be applied to sheaths having a wide variety of inner and outer diameters. Applications can utilize a sheath of the present disclosure with an inner diameter of the inner polymeric layer **68** that is expandable to an expanded diameter of from about 3 Fr to about 26 Fr, including exemplary values of about 5 Fr, about 8 Fr, about 10 Fr, about 12 Fr, about 15 Fr, about 18 Fr, about 20 Fr, about 22 Fr, about 25 Fr. The expanded diameter can vary slightly along the length of the sheath **66.** For example, the expanded outer diameter at the proximal end of the sheath **66** can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, about 25 Fr, while the expanded outer diameter at the distal end of the sheath **66** can range from about 3 Fr to about 25 Fr, including exemplary values of about 8 Fr, about 10 Fr, about 12 Fr, about 15 Fr, about 18 Fr, about 20 Fr, and about 22 Fr. In some aspects, the sheath **66** can expand to an expanded outer diameter that is from about 10% greater than the original unexpanded outer diameter to about 100% greater than the original unexpanded outer diameter, including exemplary values of about 15 % greater, about 20 % greater, about 25 % greater, about 30 % greater, about 35 % greater, about 40 % greater, about 45 % greater, about 50 % greater, about 55 % greater, about 60 % greater, about 65 % greater, about 70 % greater, about 75 % greater, about 80 % greater, about 85 % greater, about 90 % greater, and about 95 % greater than the original unexpanded outer diameter.

In some aspects, the outer diameter of the sheath **66** gradually decreases from the proximal end of the sheath **66** to the distal end of the sheath **66.** For example, in one aspect, the outer diameter can gradually decrease from about 26 Fr at the proximal end to about 18 Fr at the distal end. The diameter of the sheath **66** can transition gradually across substantially the entire length of the sheath **66.** In other aspects, the transition or reduction of the diameter of the sheath **66** can occur only along a portion of the length of the sheath **66.** For example, the transition can occur along a length from the proximal end to the distal end, where the length can range from about 0.5 inches to about the entire length of sheath **66**, including any values between any two foregoing values.

Suitable materials for the inner polymeric layer **68** can have high elastic strength and include materials discussed in connection with other aspects, especially Teflon (PTFE), polyethylene (e.g., high density polyethylene), fluoropolymers, or combinations thereof. In some aspects, the inner polymeric layer **68** has a low coefficient of friction, such as a coefficient of friction of from about 0.01 to about 0.5, including exemplary values of about 0.02, about 0.03, about 0.04, about 0.05, about 0.06, about 0.07, about 0.08 about 0.09, about 0.1, about 0.2, about 0.3, and about 0.4. Some exemplary aspects of a sheath **66** comprise an inner polymeric layer **68** having a coefficient of friction of about 0.5 or less, about 0.4 or less, about 0.3 or less, about 0.2 or less, about 0.1 or less, of about 0.09 or less, about 0.08 or less, about 0.07 or less, about 0.05 or less, about 0.04 or less, about 0.03 or less, about 0.02 or less, or about 0.01 or less.

Likewise, suitable materials for the outer polymeric layer **70** can include any of the discussed above materials and other thermoplastic elastomers and/or highly elastic materials.

The Shore hardness of the outer polymeric layer **70** can be varied for different applications and aspects. Some aspects include an outer polymeric layer with a Shore hardness of from about 25A to about 80A, including exemplary values of about 30A, about 35A, about 40A, about 45A, about 50A, about 55A, about 60A, about 65A, about 70A, and about 75A, or from about 20D to about 40D, including exemplary values of about 22D, about 25D, about 28D, about 30D, about 32 D, about 35D, and about 38D. It is understood that the Shore hardness can be any value between any two foregoing values. In one aspect, the outer polymeric layer can comprise a readily available polyurethane with a Shore hardness of 72A. Yet, in other aspects, a polyethylene inner polymeric layer can be dipped in polyurethane or silicone to create the outer polymeric layer.

The sheath **66** can also include a radiopaque filler or marker, as described above. In some aspects, a distinct radiopaque marker or band can be applied to some portion of the sheath **66.** For example, a radiopaque marker can be coupled to the inner polymeric layer **68**, the outer polymeric layer **70**, and/or can be positioned in between the inner and outer polymeric layers **68, 70.**

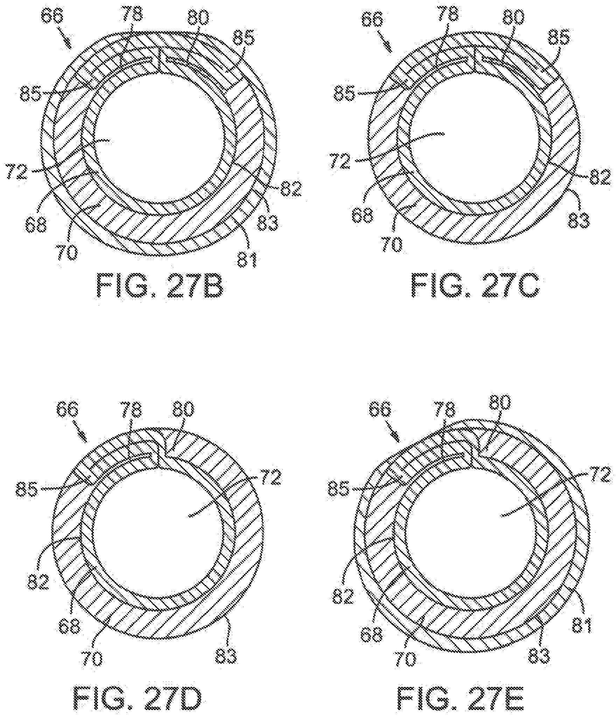

**FIGS. 27A-27E** and **28** illustrate section views of various aspects of unexpanded (**FIGS. 27A-27E**) and expanded (**FIG. 28**) sheaths **66** according to the present disclosure. The sheath **66** can include a split outer polymeric tubular layer **70** having a longitudinal cut **76** through the thickness of the outer polymeric tubular layer **70** such that the outer polymeric tubular layer **70** comprises a first portion **78** and a second portion **80** separable from one another along the cut **76.** An expandable inner polymeric layer **68** is associated with an inner surface **82** of the outer polymeric tubular layer **70**, and, in the unexpanded configuration shown in **FIG. 27A**, a portion of the inner polymeric layer **68** extends through a gap created by the cut **76** and can be compressed between the first and second portions **78, 80** of the outer polymeric tubular layer **70.** Upon expansion of the sheath **66**, as shown in **FIG. 28**, first and second portions **78, 80** of the outer polymeric tubular layer **70** have separated from one another, and the inner polymeric layer **68** is expanded to a substantially cylindrical tube. In some aspects, two or more longitudinal cuts **76** can be provided through the thickness of the outer polymeric tubular layer **70.** In such aspects, a portion of the inner polymeric layer **68** may extend through each of the longitudinal cuts **76** provided in the outer polymeric tubular layer **70.**

In certain aspects, the inner polymeric layer **68** can comprise one or more materials that are elastic and amenable to folding and/or pleating. For example, **FIG. 27A** illustrates an inner polymeric layer **68** with folded regions **85.** As seen in **FIGS. 27A-27E**, the sheath **66** can be provided with one or more folded regions **85.** Such folded regions **85** can be provided along a radial direction and substantially conform to the circumference of the outer polymeric tubular layer **70.** At least a portion of the folded regions **85** can be positioned adjacent to the outer surface **83** of the outer polymeric tubular layer **70.**

Additionally, as shown in **FIGS. 27B** and **27E**, at least a portion of the folded region or regions **85** can be overlapped by an outer covering, such as outer polymeric covering **81.** The outer polymeric covering **81** can be adjacent at least a portion of the outer surface **83** of the outer polymeric tubular layer **70.** The outer polymeric covering **81** serves to at least partially contain the folded regions **85** of the inner polymeric layer **68** and can also prevent the folded regions **85** from separating from the outer polymeric tubular layer **70** when, for example, the sheath **66** undergoes bending. In some aspects, the outer polymeric covering **81** can at least partially adhere to the outer surface **83** of the outer polymeric tubular layer **70.** The outer polymeric covering **81** can also increase the stiffness and/or durability of the sheath **66.**

Additionally, as shown in **FIGS. 27B** and **27E**, the outer polymeric covering **81** may not entirely overlap the circumference of the sheath **66.** For example, the outer polymeric covering **81** can be provided with first and second ends, where the ends do not contact one another. In these aspects, only a portion of the folded region **85** of the inner polymeric layer **68** is overlapped by the outer polymeric covering **81.**

In some aspects, where a plurality of folded regions **85** is present, the regions can be equally displaced from each other around the circumference of the outer polymeric tubular layer **70.** Alternatively, the folded regions can be off-center, different sizes, and/or randomly spaced apart from each other. In certain aspects, while portions of the inner polymeric layer **68** and the outer tubular layer **70** can be adhered or otherwise coupled to one another, the folded regions **85** are not adhered or coupled to the outer tubular layer **70.** For example, adhesion between the inner polymeric layer **68** and the outer tubular layer **70** can be highest in areas of minimal expansion.

In one exemplary and unlimiting aspect, the sheath illustrated in **FIGS. 27A-****28** can comprise a polyethylene (*e.g.*, high density polyethylene) outer polymeric tubular layer **70** and a PTFE inner polymeric layer **68.** However, other materials are suitable for each layer, as described above. In certain aspects, suitable materials for use with the outer polymeric tubular layer **70** can include materials having a high stiffness or modulus of strength that can support expansion and contraction of the inner polymeric layer **68.**

In some aspects, the outer polymeric tubular layer **70** comprises the same material or combination of materials along the entire length of the outer polymeric tubular layer **70.** In alternative aspects, the material composition can change along the length of the outer polymeric tubular layer **70.** For example, the outer polymeric tubular layer can be provided with one or more segments, where the composition changes from segment to segment. In one exemplary aspect, the Durometer rating of the composition changes along the length of the outer polymeric tubular layer **70** such that segments near the proximal end comprise a stiffer material or combination of materials, while segments near the distal end comprise a softer material or combination of materials. This can allow for a sheath **66** having a relatively stiff proximal end at the point of introducing a delivery apparatus while still having a relatively soft distal tip at the point of entry into the patient's vessel.

As with other disclosed aspects, in certain aspects, the sheath **66** shown in **FIGS. 27A-28** can be provided in a wide range of sizes and dimensions. For example, the sheath **66** can be provided with an unexpanded inner diameter of from about 3 Fr to about 26 Fr., including exemplary values of about 5 Fr, about 8 Fr, about 10 Fr, about 12 Fr, about 15 Fr, about 18 Fr, about 20 Fr, about 22 Fr, about 25 Fr. In some aspects, the sheath **66** has an unexpanded inner diameter of from about 15 Fr to about 16 Fr. In some other aspects, the unexpanded inner diameter of the sheath **66** can range from about 3 Fr to about 26 Fr, including exemplary values of about 5 Fr, about 8 Fr, about 10 Fr, about 12 Fr, about 15 Fr, about 18 Fr, about 20 Fr, about 22 Fr, about 25 Fr at or near the distal end of sheath **66**, while the unexpanded inner diameter of the sheath **66** can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, and about 25 Fr at or near the proximal end of sheath **66.** For example, in one aspect, the sheath 66 can transition from an unexpanded inner diameter of about 16 Fr at or near the distal end of the sheath **66** to an unexpanded inner diameter of about 26 Fr at or near the proximal end of the sheath **66.**

**The** sheath **66** can be provided with an unexpanded outer diameter of from about 3 Fr to about 30 Fr, including exemplary values of about 5 Fr, about 8 Fr, about 10 Fr, about 12 Fr, about 15 Fr, about 18 Fr, about 20 Fr, about 22 Fr, about 25 Fr, and about 28 Fr. Yet in other aspects, it can have an unexpanded outer diameter of from about 18 Fr to about 19 Fr. In some aspects, the unexpanded outer diameter of the sheath **66** can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, and about 25 Fr at or near the distal end of sheath **66**, while the unexpanded outer diameter of the sheath 66 can range from about 3 Fr to about 30 Fr, including exemplary values of about 5 Fr, about 8 Fr, about 10 Fr, about 12 Fr, about 15 Fr, about 18 Fr, about 20 Fr, about 22 Fr, about 25 Fr, and about 28 Fr at or near the proximal end of sheath **66.** For example, in one aspect, the sheath **66** can transition from an unexpanded outer diameter of about 18 Fr at or near the distal end of the sheath **66** to an unexpanded outer diameter of about 28 Fr at or near the proximal end of the sheath **66.**

In some exemplary aspects, the thickness of the inner polymeric layer **68** can vary from about 0.002 inches to about 0.015 inches, including exemplary values of about 0.003, about 0.004, about 0.005, about 0.006, about 0.007, about 0.008, about 0.009, about 0.01, and about 0.012 inches. In other aspects, expansion of the sheath **66** can result in an expansion of the unexpanded outer diameter of from about 10% or less to about 430% or more. In certain aspects, expansion of the sheath **66** can result in expansion of the unexpanded outer diameter to about 10 % or less, to about 9 % or less, to about 8 % or less, to about 7 % or less, to about 6 % or less, to about 5 % or less, to about 4 % or less, to about 3 % or less, to about 2 % or less, to about 1 % or less. In yet other aspects, expansion of the sheath **66** can result in expansion of the unexpanded outer diameter to about 10 % or more, about 20 % or more, about 30 % or more, about 40 % or more, about 50 % or more, about 60 % or more, about 70 % or more, about 80 % or more, about 90 % or more, about 100 % or more, about 125 % or more, about 150 % or more, about 175 % or more, about 200 % or more, about 225 % or more, about 250 % or more, about 275 % or more, about 300 % or more, about 325 % or more, about 350 % or more, about 375 % or more, about 400 % or more, or about 425 % or more, about 450 % or more, about 475 % or more, or about 500 % or more. As with other illustrated and described aspects, the sheaths shown in **FIGS. 27A-28** can be provided with a radiopaque filler and/or a radiopaque tip marker, as described above. The sheath **66** can be provided with a radiopaque tip marker provided at or near the distal tip of the sheath **66.** Such a radiopaque tip marker can comprise materials such as those suitable for the radiopaque filler, platinum, iridium, platinum/iridium alloys, stainless steel, other biocompatible metals, or combinations thereof.

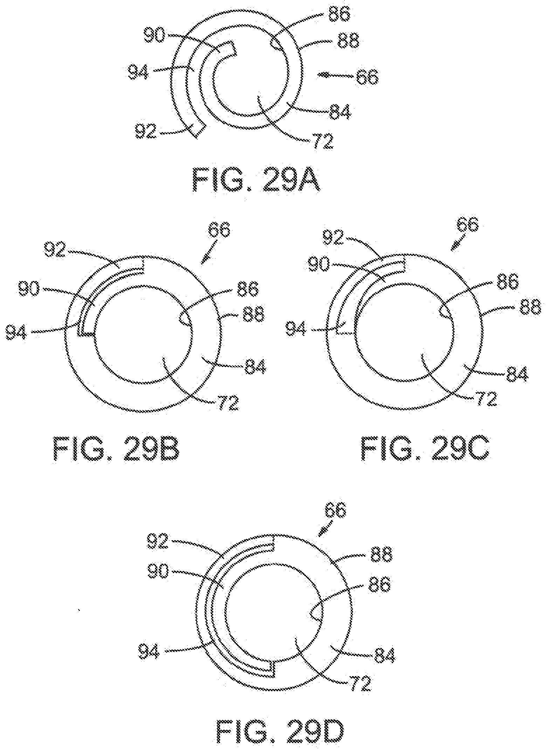

**FIGS. 29A-29D** show section views of other possible configurations of a sheath **66** for introducing a prosthetic device into a patient's vasculature. The sheath **66** comprises a polymeric tubular layer **84** having an inner surface **86** and an outer surface **88.** The thickness of the polymeric tubular layer **84** extends from the inner surface **86** to the outer surface **88.** As shown in **FIGS. 29B-29D**, the polymeric tubular layer **84** can be formed with at least a first angular portion **90** of reduced thickness adjacent the inner surface **86** and a second angular portion **92** of reduced thickness adjacent to the outer surface **88**, with the second portion **92** at least partially overlapping the first portion **90.** **FIG. 29A** illustrates a similar configuration, where a second portion **92** at least partially overlaps a first portion **90** in a partial coil configuration. In some aspects and as shown in **FIG. 29A**, the second portion **92,** and the first portion **90** of the sheath can have the same thickness.

In some exemplary aspects, the first and second portions **90, 92** are not adhered to one another. In some aspects, and as seen in **FIG. 29A**, there can be a small gap **94** between the first and second portions **90, 92** that can give the sheath **66** the appearance of having two interior lumens **72, 94.** **FIGS. 29A-29D** illustrate the sheath **66** in unexpanded configurations. In certain aspects, upon expansion of the sheath **66**, the ends of the first and second portions **90, 92** abut or are in close proximity to each other to reduce or eliminate any gap between them.

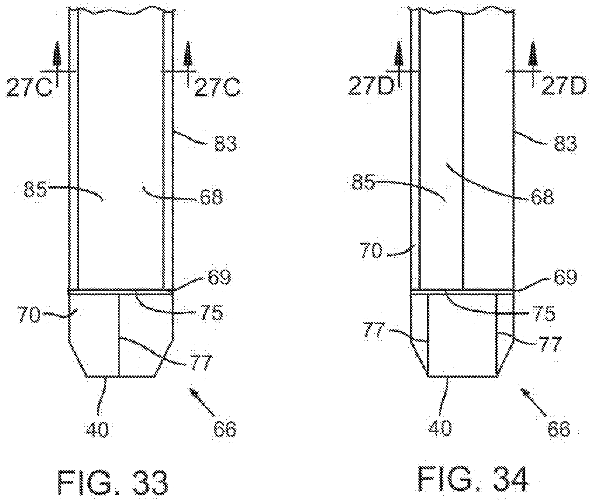

In some aspects, a sheath **66** can comprise a partial slit or scoreline along at least a portion of its length. For example, as shown in **FIG. 33**, a sheath **66** can comprise an outer polymeric tubular layer **70** over an inner polymeric layer **68.** The inner polymeric layer can extend through a cut in the outer polymeric tubular layer **70** to form a folded region **85** on the outer surface of the outer polymeric tubular layer **70**, such as also shown in **FIG. 27C****.** The folded region **85** of the inner layer, in some aspects, terminates before the outer polymeric tubular layer **70** (*i.e.*, the outer polymeric tubular layer **70** is longer than the inner layer). As shown in **FIG. 33**, in these aspects, the sheath **66** can comprise a partial slit or scoreline **77** that can extend from the termination (distal end) **75** of the folded region **85** to the distal end **40** of the sheath **66.** In some aspects, scoreline **77** can facilitate expansion of the sheath **66.**

Scoreline **77** can be substantially centrally located with respect to the folded region **85.** In alternative aspects, scoreline **77** can be positioned in other locations relative to the folded region **85.** Also, sheath **66** can comprise one or more scorelines **77.** For example, as shown in **FIG. 34**, one or more scorelines **77** can be peripherally located with respect to the folded region **85.** The one or more scorelines **77** can be positioned anywhere around the circumference of the outer polymeric tubular layer **70.** In aspects comprising a radiopaque marker **69** as seen in **FIG. 33**, a scoreline **77** can extend from, for example, the distal end of the radiopaque marker **69** substantially to the distal end **40** of the sheath **66.**

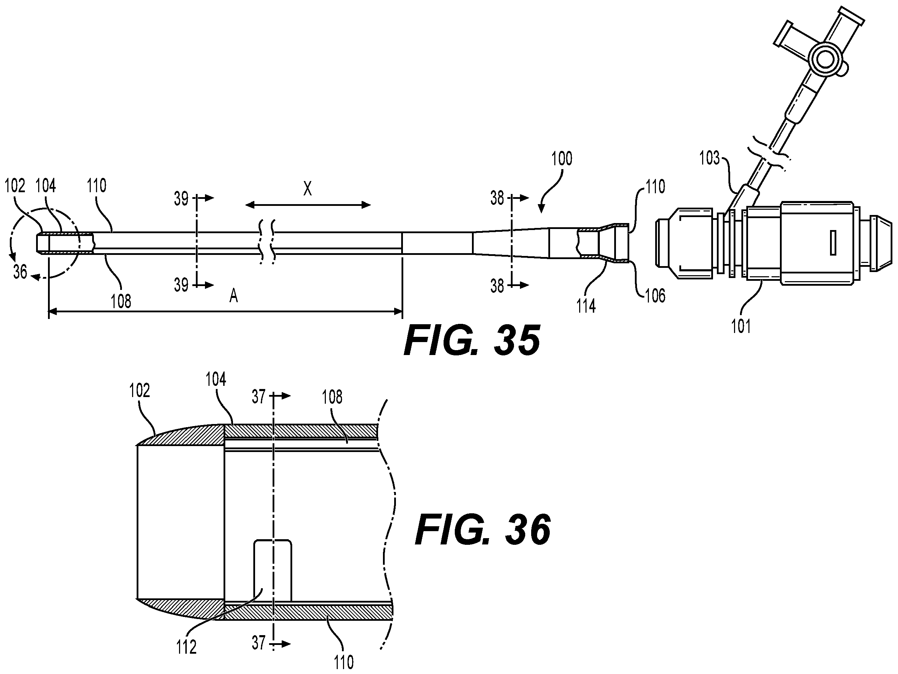

**FIGS. 35** and **36** illustrate an expandable sheath **100** according to the present disclosure, which can be used with a delivery apparatus for delivering a prosthetic device, such as a tissue heart valve into a patient. In general, the delivery apparatus can include a steerable guide catheter (also referred to as a flex catheter) a balloon catheter extending through the guide catheter, and a nose catheter extending through the balloon catheter (*e.g.*, as depicted in **FIG. 1**). The guide catheter, the balloon catheter, and the nose catheter can be adapted to slide longitudinally relative to each other to facilitate delivery and positioning of the valve at an implantation site in a patient's body. However, it should be noted that the sheath **100** can be used with any type of elongated delivery apparatus used for implanting balloon-expandable prosthetic valves, self-expanding prosthetic valves, and other prosthetic devices. Generally, sheath **100** can be inserted into a vessel (*e.g.*, the femoral or iliac arteries) by passing through the skin of a patient, such that a soft tip portion **102** at the distal end **104** of the sheath **100** is inserted into the vessel. The sheath **100** can also include a proximal flared end portion **114** to facilitate mating with an introducer housing **101** and catheters mentioned above (*e.g.*, the proximal flared end portion **114** can provide a compression fit over the housing tip and/or the proximal flared end portion 114 can be secured to the housing **101** via a nut or other fastening device or by bonding the proximal end of the sheath to the housing). The introducer housing **101** can house one or more valves that form a seal around the outer surface of the delivery apparatus once inserted through the housing, as known in the art. The delivery apparatus can be inserted into and through the sheath **100**, allowing the prosthetic device to be advanced through the patient's vasculature and implanted within the patient.

Sheath **100** can include a plurality of layers. For example, sheath **100** can include an inner layer **108** and an outer layer **110** disposed around the inner layer **108.** The inner layer **108** can define a lumen through which a delivery apparatus can travel into a patient's vessel in order to deliver, remove, repair, and/or replace a prosthetic device, moving in a direction along the longitudinal axis X. As the prosthetic device passes through the sheath **100**, the sheath locally expands from a first, resting diameter to a second, expanded diameter to accommodate the prosthetic device. After the prosthetic device passes through a particular location of the sheath **100**, each successive expanded portion or segment of the sheath **100** at least partially returns to the smaller, resting diameter. In this manner, the sheath **100** can be considered self-expanding in that it does not require the use of a balloon, dilator, and/or obturator to expand.

The inner and outer layers **108, 110** can comprise any suitable materials. Suitable materials for the inner layer **108** include, but are not limited to, polytetrafluoroethylene (PTFE), ethylene tetrafluoroethylene (ETFE), nylon, polyethylene, polyether block amide (*e.g.*, Pebax), and/or combinations thereof. In one exemplary aspect, the inner layer **108** can comprise a lubricious, low-friction, or hydrophilic material, such as PTFE. Such a low coefficient of friction materials can facilitate passage of the prosthetic device through the lumen defined by the inner layer **108.** In some aspects, the inner layer **108** can have a coefficient of friction of less than about 0.5, of less than about 0.4, of less than about 0.3, of less than about 0.2, of less than about 0.1, or less than about 0.09, or less than about 0.08, or less than about 0.07. or less than about 0.05. In some aspects, the sheath **100** can include a lubricious liner on the inner surface of the inner layer **108.** Examples of suitable lubricious liners can include materials that can further reduce the coefficient of friction of the inner layer **108**, such as, for example, and without limitations, PTFE, polyethylene, polyvinylidene fluoride, and combinations thereof. Suitable materials for a lubricious liner also include other materials desirably having a coefficient of friction of about 0.5 or less, about 0.4 or less, about 0.3 or less, about 0.2 or less, about 0.1 or less, about 0.09 or less, about 0.08 or less, about 0.07 or less, about 0.06 or less, or about 0.05 or less.

Suitable materials for the outer layer **110** can include nylon, polyethylene, Pebax, HDPE, polyurethanes (*e.g.*, Tecoflex), and other medical-grade materials. In one aspect, the outer layer **110** can comprise high density polyethylene (HDPE) and Tecoflex (or other polyurethane material) extruded as a composite. In some aspects, the Tecoflex can act as an adhesive between the inner layer **108** and the outer layer **110** and may only be present along a portion of the inner surface of the outer layer 110. Other suitable materials for the inner and outer layers are also disclosed in U.S. Patent Application Publication No. 2010/0094392.

Additionally, in some aspects, the sheath **100** can include an exterior hydrophilic coating on the outer surface of the outer layer **110.** Such a hydrophilic coating can facilitate insertion of the sheath **100** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g.*, PTFE, polyethylene, polyvinylidene fluoride), are also suitable for use with the sheath **100.**

Best seen in **FIG. 36**, the soft tip portion **102** can comprise, in some aspects, low density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 D to about 40 D, including exemplary values of about 28 D, about 30 D, about 32 D, about 35 D, and about 38 D. It is further understood that Shore hardness can have any value between any two foregoing values. The tip portion **102** can be configured to be radially expandable to allow a prosthetic device to pass through the distal opening of the sheath **100.** For example, the tip portion **102** can be formed with a weakened portion, such as an axially extending scoreline or perforated line that is configured to split and allow the tip portion to expand radially when the prosthetic device passes through the tip portion (such as shown in the aspects of **FIGS. 33** and **34****).**

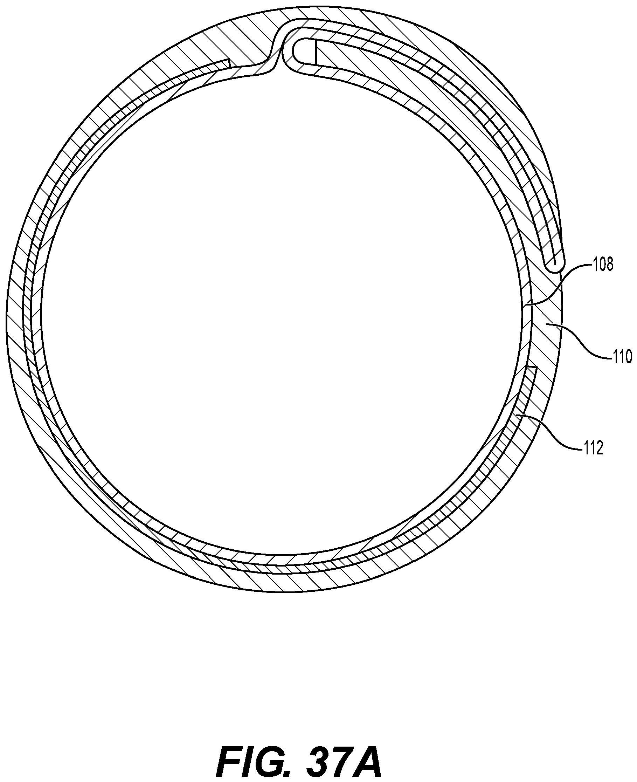

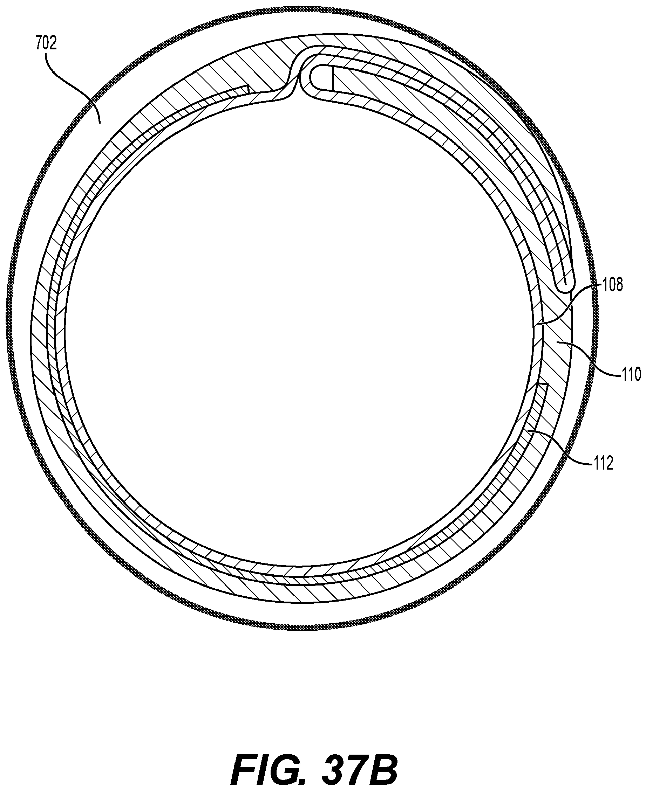

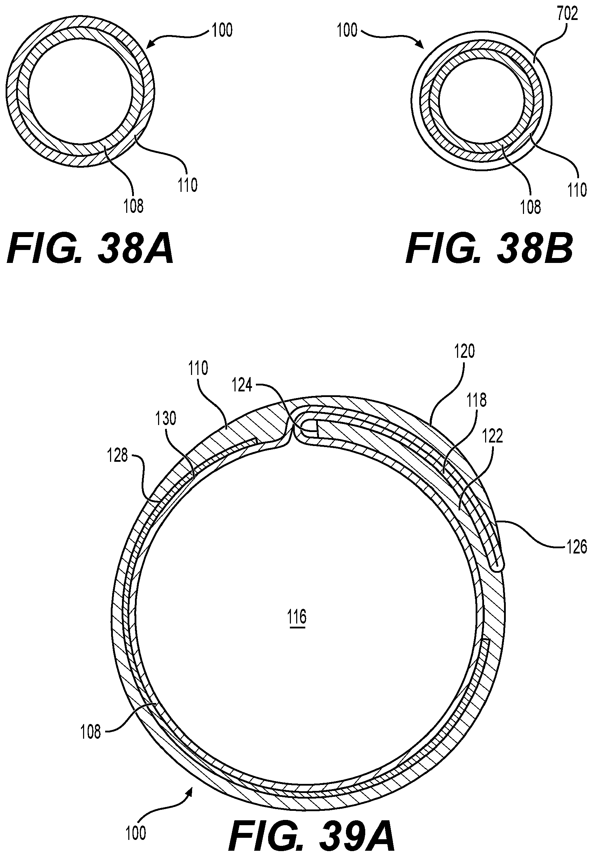

**FIG. 37** shows a cross-section view of the sheath **100** taken near the distal end **104** of the sheath **100,** in one aspect. As shown in **FIGS. 36** and 37A-B, the sheath **100** can include at least one radiopaque filler or marker, such as a discontinuous, or C-shaped, band **112** positioned near the distal end **104** of the sheath **100.** The marker **112** can be associated with the inner and/or outer layers **108, 110** of the sheath **100.** For example, as shown in **FIG. 37A**, the marker **112** can be positioned between the inner layer **108** and the outer layer **110.** In alternative aspects, the marker **112** can be associated with the outer surface of the outer layer **110.** In some aspects, the marker **112** can be embedded or blended within the inner or outer layers **108**, **110.** In a still further aspect, the sheath can comprise an outer jacket or an outer cover as described herein. Such exemplary aspects are shown in **FIG. 37B****,** wherein the outer jacket **702** (in some aspects, the outer jacket is an elastic cover) is present. It is understood that the outer jacket can be present along all the length of the sheath, including the distal and proximal end of the sheath (**FIG**. **37B** and **FIG. 38B**). In yet other aspects, the outer jacket can only be present along a portion of the sheath. It is further understood that the portion of the sheath can comprise any location along the sheath's length.

In certain aspects, wherein the outer jacket is present, it can comprise an inner surface and outer surface. In such aspects, the outer jacket extends at least partially around the outer layer such that the inner surface of the outer jacket overlies the outer surface of the outer layer. In yet further aspects, the outer jacket overlies the outer surface of the outer layer along a circumference of the outer layer along its longitudinal axis.

The C-shaped band **112** can serve as a radiopaque marker or filler to enable visibility of the sheath **100** under fluoroscopy during use within a patient. The C-shaped band **112** can comprise any suitable radiopaque material, such as barium sulfite, bismuth trioxide, titanium dioxide, bismuth subcarbonate, platinum, iridium, and combinations thereof. In one exemplary and unlimiting aspect, the C-shaped band can comprise 90% platinum and 10% iridium. In other aspects, the marker **112** needs not to be a C-shaped band. Other shapes, designs, and configurations are possible. For example, in some aspects, the marker **112** can extend around the entire circumference of the sheath **100.** In other aspects, the marker **112** can comprise a plurality of small markers spaced around the sheath **100.**

In certain aspects, disclosed herein is a sheath for delivering a medical device, wherein the sheath has a proximal and a distal end and comprises: an expandable inner layer having an inner surface and an outer surface, wherein the inner surface of the expandable inner layer defines a lumen having a longitudinal axis and comprising at least one folded portion having an inner portion and outer portion; an outer layer having an inner surface and an outer surface and extending at least partially around the inner layer such that at least a first portion of the outer surface of the outer layer is positioned adjacent to the inner portion of the at least one folded portion of the inner layer, while a first portion of the inner surface of the outer layer is positioned adjacent to the outer portion of the at least one folded portion of the inner layer; a first polymer disposed between at least a portion of the inner layer and at least a portion of the outer layer, forming an intermediate layer, wherein, the first polymer exhibits a melting temperature from about 30 °C to about 45 °C; and wherein the at least one folded portion is configured to at least partially unfold during application of a radial outward force by passage of a medical device through the lumen of the inner layer. In still further aspects, the first polymer can be disposed between at least the first portion of the outer surface of the outer layer and the inner portion of the at least one folded portion of the inner layer, forming an intermediate layer between the inner portion of the at least one folded portion and the at least first portion of the outer surface of the inner layer. In still further aspects, the first polymer can be disposed between at least the first portion of the outer surface of the outer layer and the inner portion of the at least one folded portion of the inner layer.

In some aspects, the first polymer can be disposed between at least the first portion of the inner surface of the outer layer and the outer portion of the at least one folded portion of the inner layer. In still further aspects, the sheath can further comprise an outer jacket comprising an inner surface and outer surface extending at least partially around the outer layer such that the inner surface of the outer jacket overlies the outer surface of the outer layer. In a certain aspect, the first polymer is disposed between at least a portion of the inner surface of the outer jacket and at least a portion of the outer surface of the outer layer.

In still further aspects, the first polymer can be disposed between the first portion of the outer surface of the outer layer and the inner portion of the at least one folded portion of the inner layer along a longitudinal axis of the folded portion. In still other aspects, the first polymer can be disposed along a circumference of the outer surface of the outer layer along its longitudinal axis. FIGS. 38A-B and 39A-B show additional cross sections taken at different points along the sheath **100.** **FIG. 38A** shows a cross-section of a segment of the sheath near the proximal end **106** of the sheath **100**, as indicated by a line **38-38** in **FIG. 35** without the presence of the outer jacket **702**, when **FIG. 38B** shows a cross-section of a segment of the sheath near the proximal end **106** of the sheath **100**, as indicated by line **38-38** in **FIG. 35** when the outer jacket **702** is present. In certain aspects where the outer jacket is present, the outer surface of the outer layer of the disclosed sheath can comprise a second portion configured to seal with at least a first portion of the inner surface of the outer jacket at at least a portion of the distal end of the sheath.

In yet other aspects where the outer jacket is present, the outer surface of the outer layer of the disclosed sheath comprises a third portion configured to seal with at least a second portion of the inner surface of the outer jacket at at least a portion of the proximal end of the sheath.

The sheath **100** at the location shown in FIGs 38A-B can include inner layer **108** and outer layer **110.** At this location, near the proximal end of the sheath, the layers **108**, **110** can be substantially tubular, without any slits or folded portions in the layers. By contrast, the layers **108, 110** at different locations along the sheath **100** (*e.g.*, at the point indicated by line **39-39** in **FIG. 35**) can have a different configuration.

**FIG. 39A** shows a cross section of the sheath without the presence of the first polymer. As shown in **FIG. 39A**, the inner layer **108** can be arranged to form a substantially cylindrical lumen **116** therethrough. Inner layer **108** can include one or more folded portions **118.** In the aspect shown in **FIG. 39A**, inner layer **108** is arranged to have one folded portion **118** that can be positioned on either side of the inner layer **108.** Inner layer **108** can be continuous in that there are no breaks, slits, or perforations in inner layer **108.** Outer layer **110** can be arranged in an overlapping fashion such that an overlapping portion **120** overlaps at least a part of the folded portion **118** of the inner layer **108.** As shown in **FIG. 39A**, the overlapping portion **120** also overlaps an underlying portion **122** of the outer layer **110.** The underlying portion **122** can be positioned to underlie both the overlapping portion **120** of the outer layer **110**, as well as the folded portion **118** of the inner layer **108.** Thus, the outer layer **110** can be discontinuous, including a slit or a cut in order to form the overlapping and underlying portions **120, 122.** In other words, a first edge **124** of the outer layer **110** is spaced apart from a second edge **126** of the outer layer **110** so as not to form a continuous layer.

In some aspects, the sheath can further comprise a tie layer disposed between at least a second portion of the inner surface of the outer layer and at least a portion of the outer surface of the inner layer, such that it is configured to seal the at least a second portion of the inner surface of the outer layer with the at least a portion of the outer surface of the inner layer. In yet other aspects, the tie layer is disposed between the inner surface of the outer layer and the outer surface of the inner layer along a circumference of the inner layer along its longitudinal axis.

As shown in **FIG. 39A**, the sheath **100** can optionally include a thin layer of bonding or adhesive material (a tie layer) **128** positioned between the inner and outer layers **108, 110.** In one aspect, the adhesive material **128** can comprise a polyurethane material such as Tecoflex, or polymer, copolymer, or terpolymer such as maleic anhydride modified polyolefin, for example, and without limitation, Orevac^{®} (commercially available from Arkema), ethylene acrylic acid copolymer, such as DOW Chemical Primacor^{®}, ethylene acrylate copolymer such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymer such as Lotader^{®} or Orevac^{®} (commercially available from Arkema). In certain aspects, the adhesive material (tie layer) **128** can be positioned on an inner surface **130** (at least a second portion of the inner surface) of at least a portion of the outer layer **110** so as to provide adhesion between selected portions of the inner and outer layers **108, 110.** In such exemplary aspects, the tie layer can be configured to seal the at least a second portion of the inner surface of the outer layer with at least a portion of the inner layer. For example, the outer layer **110** can only include a tie layer **128** around the portion of the inner surface **130** that faces the lumen-forming portion of the inner layer **108.** In other words, in some aspects, the tie layer **128** can be positioned so that it does not contact the folded portion **118** of the inner layer **108.** In other aspects, the tie layer **128** can be positioned in different configurations as desired for that particular application. For example, as shown in **FIG. 39A**, the tie layer **128** can be positioned along the entire inner surface **130** of the outer layer **110.** In an alternative aspect, the tie layer can be applied to the outer surface of the inner liner **108** instead of the inner surface of the outer layer. The tie layer can be applied to all or selected portions of the inner layer; for example, the tie layer can be formed only on the portion of the inner layer that faces the lumen-forming portion of the outer layer and not on the folded portion. The configuration of **FIG. 39A** allows for radial expansion of the sheath **100** as an outwardly directed radial force is applied from within (*e.g.*, by passing a medical device such as a prosthetic heart valve through the lumen **116**). As radial force is applied, the folded portion **118** can at least partially separate, straighten, and/or unfold, and/or the overlapping portion **120** and the underlying portion **122** of the outer layer **110** can slide circumferentially with respect to one another, thereby allowing the diameter of lumen **116** to enlarge.

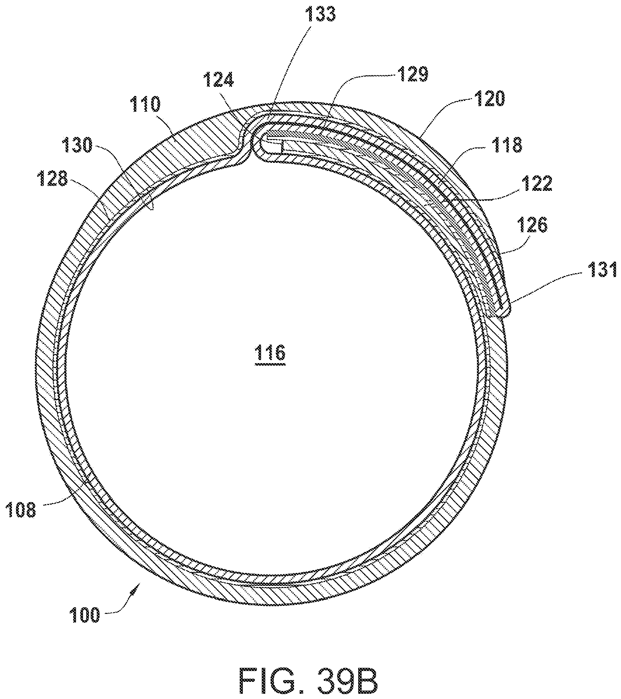

**FIG. 39B** shows a cross-section of the sheath comprising a first polymer disposed between the at least the first portion of the outer surface of the outer layer **110** and the inner portion of the at least one folded portion of the inner layer **118**, forming an intermediate layer **129** between the inner portion of the at least one folded portion and the at least first portion of the outer surface of the outer layer.

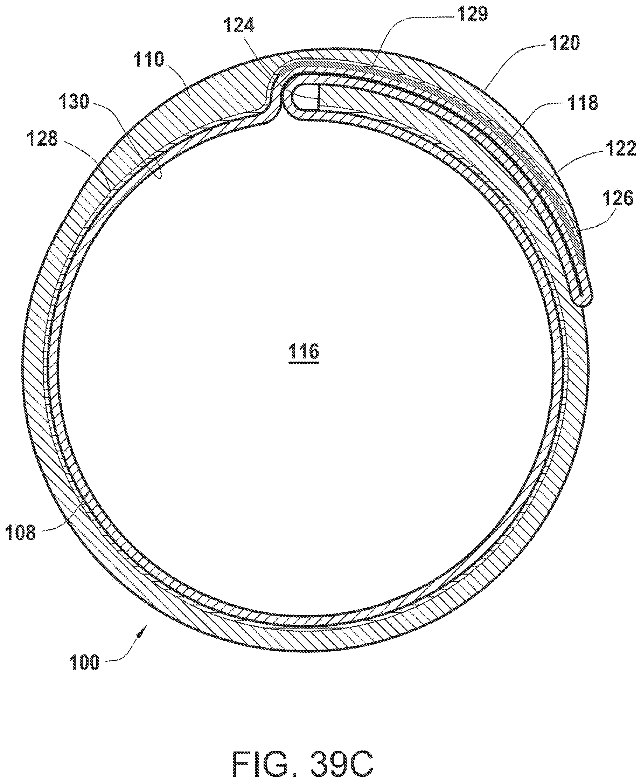

It is, however, understood that the location of the first polymer is not limited by the exemplary sheath shown in **FIG. 39B****.** Additionally, exemplary locations for the first polymer are shown in FIG. 39C-G. For example, **FIG. 39C** shows the exemplary sheath comprising the first polymer **129** disposed between the outer layer **110** and the folded portion **118** of the inner layer **108.** In some exemplary aspects, the first polymer **129** can be disposed between an outer portion of the folded portion **118** and at least a portion of the inner surface **130** of the outer layer **110** that overlaps the folded portion.

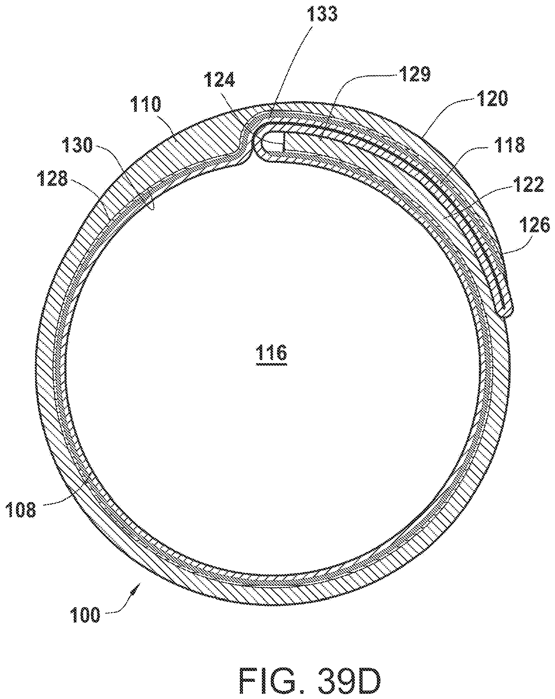

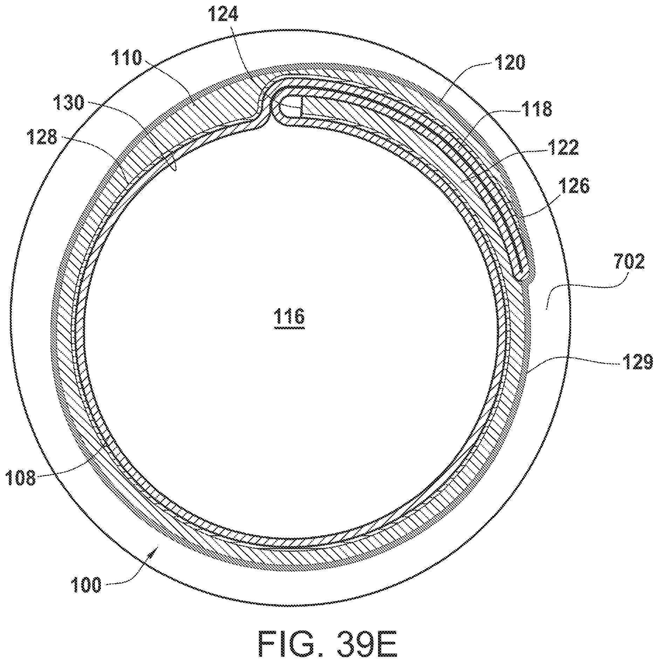

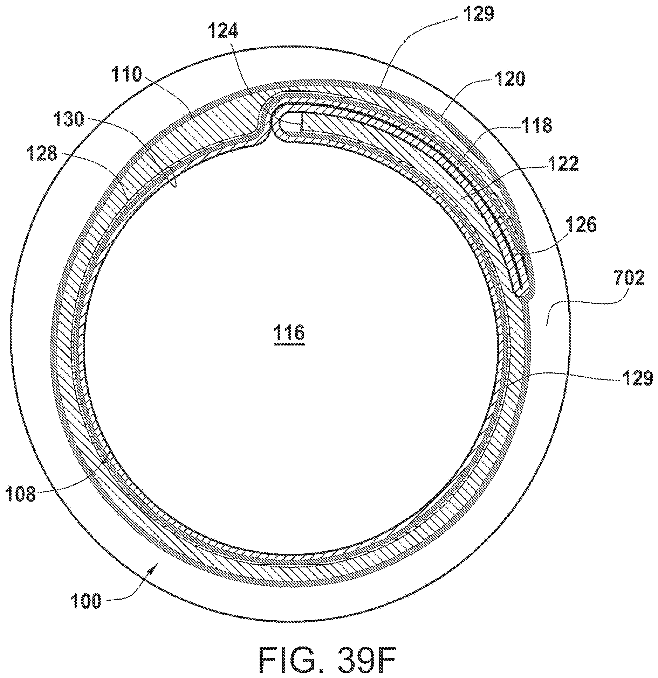

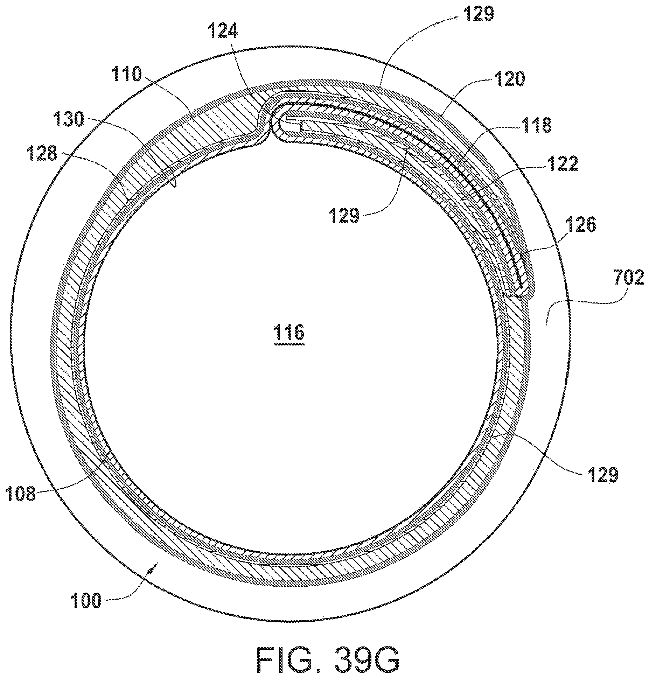

In yet other aspects, and as shown in **FIG. 39D****,** the exemplary sheath can comprise the first polymer **129** disposed circumferentially between the inner layer **108** and outer layer **110.** For example, as provided in **FIG. 39D**, the first polymer **129** can be disposed between the outer layer **110** and the folded portion **118** of the inner layer **108** and continue circumferentially between the outer layer **110** and the inner layer **108** (up to the second longitudinally extending edge **124**). In some aspects, as shown in **FIG. 39E****,** when the outer jacket **702** is present in the sheath **100**, the first polymer **129** can be disposed between the outer surface of the outer layer **110** and the inner surface of the outer jacket **702.** For example, the first polymer **129** can extend around all, or a portion, of the circumference of the outer surface of the outer layer **110.** **FIG. 39F** shows aspects where the outer jacket **702** is present, and the first polymer **129** can be disposed between the inner surface of the outer jacket **702** and the outer surface of the outer layer **110**, and between the outer layer **110** and the inner layer **108** (i.e., between the outer layer **110** and the folded portion **118** of the inner layer **108**, and continue circumferentially between the outer layer **110** and the inner layer **108**). As illustrated in **FIG. 39F**, the first polymer **129** can be disposed between the inner surface of the outer layer **110** and the outer surface of the inner layer **108** around the entire circumference of the sheath. **FIG. 39G** shows an exemplary configuration that is similar to one disclosed in **FIG. 39F** with the additional first polymer **129** disposed between at least the first portion of the outer surface of the outer layer **110** and the inner portion/underside of the folded portion **118** of the inner layer **108**, forming an intermediate layer **129** between the inner portion of the at least one folded portion **118** and the at least first portion of the outer surface of the outer layer **110.**

In another aspect, the folded portion of the disclosed sheath and as shown in **FIG. 39B** can comprise a first folded edge **131** and a second folded edge **133** and an overlapping portion extending circumferentially between the first and second folded edges, the overlapping portion comprising an overlap in a radial direction of at least two thicknesses of the inner layer, wherein the first folded edge **131** is configured to move closer to the second folded edge **133** to shorten the overlapping portion at a local axial location during application of a radial outward force by passage of the medical device and wherein shortening of the overlapping portion corresponds with a local expansion of the lumen, and wherein the first polymer is provided between the at least the first portion of the outer surface of the outer layer **110** and an inner surface of the overlapping portion of the folded portion.

Still further, the folded portion can comprise a first folded edge **131** and a second folded edge **133** and an overlapping portion extending circumferentially between the first and second folded edges, the overlapping portion comprising an overlap in a radial direction of at least two thicknesses of the inner layer, wherein the first folded edge is configured to move closer to the second folded edge to shorten the overlapping portion at a local axial location during application of a radial outward force by passage of the medical device and wherein shortening of the overlapping portion corresponds with a local expansion of the lumen, the overlapping portion, wherein the outer layer includes a first longitudinally extending edge **126** and a second longitudinally extending edge **124** configured to separate as the sheath expands, the first longitudinal extending edge and an overlapping portion of the outer layer extending over the second longitudinal extending edge when the sheath is not expanded, and wherein the first polymer is provided between the at least the first portion of the outer surface of the outer layer proximate the second longitudinally extending edge and the inner surface of the overlapping portion of the folded portion.

In still further aspects, the sheath as shown in **FIGs. 39B-39G** can also comprise a tie layer **128.** It is understood that the tie layer can be optional, and the aspects where the sheath does not comprise the tie layer are also disclosed. The tie layer can comprise any material disclosed above, for example, and without limitation, it can comprise a polyurethane material such as Tecoflex, or polymer, copolymer, or terpolymer such as maleic anhydride modified polyolefin, for example, and without limitation, Orevac^{®} (commercially available from Arkema), ethylene acrylic acid copolymer, such as DOW Chemical Primacor^{®}, ethylene acrylate copolymer such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymer such as Lotader^{®} or Orevac^{®} (commercially available from Arkema).

In still further aspects, the inner layer can comprise any known in the art and/or disclosed herein, polymers. In still further aspects, the inner layer can comprise polytetrafluoroethylene (PTFE), polyimide, polyetheretherketone (PEEK), polyurethane, nylon, polyethylene, high density polyethylene (HDPE) polyamide, or combinations thereof.

In still further aspects, the expandable inner layer can have a dynamic coefficient of friction of less than about 0.5 against steel as measured according to ASTM D1894, including exemplary values of less than about 0.4, less than about 0.3, less than about 0.2, less than about 0.1, less than about 0.09, less than about 0.08, less than about 0.07, less than about 0.06, less than about 0.05, less than about 0.04, less than about 0.03, less than about 0.02, or less than about 0.01 as measured according to ASTM D1894.

In some of the aspects disclosed herein, the outer layer can comprise PTFE, polyimide, PEEK, polyurethane, nylon, polyethylene, polyamide, polyether block amides, polyether block ester copolymer, thermoset silicone, latex, poly-isoprene rubbers, high density polyethylene (HDPE), Tecoflex, or combinations thereof. In still further aspects, the outer surface of the expandable inner layer can be etched. It is understood that the etched surface can be obtained by any known in the art methods to arrive at the desired results. In certain aspects, the etching can be a solution-based etch or a plasma etch and/or laser ablation.

In one exemplary aspect, the inner layer can comprise PTFE, and the outer layer can comprise a combination of HDPE and Tecoflex. In still other aspects, the inventive sheath can optionally comprise an elastic outer cover. If present, the elastic cover can include any suitable materials, such as any suitable heat shrink materials. Examples include Pebax, polyurethane, silicone, and/or polyisoprene.

In still further aspects, the inner surface of the outer layer comprises a third portion configured to seal with the outer surface of the inner layer at at least a portion of the distal end of the sheath. In yet other aspects, the inner surface of the outer layer can comprise a fourth portion configured to seal with the outer surface of the inner layer at at least a portion of the proximal end of the sheath. In still further aspects, and as described above, when the outer jacket is present, the outer surface of the outer layer can comprise a second portion configured to seal with at least a first portion of the inner surface of the outer jacket at at least a portion of the distal end of the sheath. In still further aspects and when the outer jacket is present, the outer surface of the outer layer can comprise a third portion configured to seal with at least a second portion of the inner surface of the outer jacket at at least a portion of the proximal end of the sheath.

In still further aspects, the first polymer can have a melting temperature between about 30 °C to about 45 °C, including exemplary values of about 31 °C, about 32 °C, about 33 °C, about 34 °C, about 35 °C, about 36 °C, about 37 °C, about 38 °C, about 39 °C, about 40 °C, about 41 °C, about 42 °C, about 43 °C, and about 44 °C. In still further aspects, the first polymer is solid at a temperature below 30 °C. It is understood that in such aspects, when the sheath is present in the environment at a temperature of below 30 °C, the first portion of the outer surface of the outer layer of the sheath is adhered to the at least one folded portion of the inner layer. In still further aspects, the first polymer is disposed along a circumference of the inner layer along its longitudinal axis.

In still further aspects where the sheath is present in the environment at a temperature from about 30 °C to about 45 °C, i.e., within the melting temperature of the first polymer, the intermediate layer can become viscoelastic fluid and can behave as a lubricant between the folded portion and the outer layer. In such aspects, when the sheath is inserted into a body having a temperature of 37 °C, the viscoelastic intermediate layer can behave as a lubricant allowing a smooth expansion of the sheath. In further aspects, the sheath can exhibit at least about a 5 % reduction in insertion force when inserted into a body having a temperature of 37 °C when compared to a substantially identical reference sheath without the presence of the first polymer. Here is it understood that the insertion force relates to the force needed to insert any delivery apparatus described herein within the body. In still further exemplary aspects, the disclosed sheath having the first polymer disposed as described can exhibit at least about 10 %, at least about 15 %, at least about 20 %, at least about 25 %, at least about 30 %, at least about 35 %, at least about 40 %, at least about 45 %, at least about 50 % reduction in insertion force.

In still further aspects, the sheath can require an insertion force that is at least similar to insertion force when other adhesives, such as, for example, NuSil^{®}, are present. Yet, in other aspects, the sheath requires a reduced insertion force when compared to the sheath having an adhesive, such as, for example, NuSil^{®}. In further aspects, the sheath can exhibit at least about a 5 % reduction in insertion force when inserted into a body having a temperature of 37 °C when compared to a substantially identical reference sheath having NuSil^{®} as an adhesive. In still further exemplary aspects, the disclosed sheath having the first polymer disposed as described can exhibit at least about 10 %, at least about 15 %, at least about 20 %, at least about 25 %, at least about 30 %, at least about 35 %, at least about 40 %, at least about 45 %, at least about 50 % reduction in insertion force in the comparable sheath with the presence of different adhesive materials. It is also understood that when compared with the other commonly used adhesives, the presence of the disclosed herein polymers is preferable as they can melt in the body and do not require any additional preparations. Other adhesives, such as for example, NuSil^{®}, on the other hand, require extensive processing steps, for example, curing for at least 30 min at 90 °C that is both inconvenient and costly.

In still further aspects, when the outer jacket is present, the disclosed sheath can comprise a second polymer and/or a third polymer. In such aspects, the second polymer can exhibit a melting temperature above about 45 °C and can be disposed between the second portion of the outer surface of the outer layer at at least a portion of the distal end of the sheath and the at least a first portion of the inner surface of the outer jacket. It is understood that the difference in the melting point allows the second polymer to stay solid while the first polymer becomes a viscoelastic fluid when the sheath is inserted into a body at a temperature of 37 °C. In aspects where the second polymer is solid, it allows the second portion of the outer surface of the outer layer at at least a portion of the distal end of the sheath and the at least a first portion of the inner surface of the outer jacket and prevents the first polymer from being leached out of the sheath into the surrounding environment.

In yet further aspects, the third polymer, when present in the disclosed sheath, can exhibit a melting temperature above about 45 °C and it can be disposed between the third portion of the outer surface of the outer layer at at least a portion of the proximal end of the sheath and the at least a second portion of the inner surface of the outer jacket. It is understood that, again, the difference in the melting points allows the third polymer to stay solid while the first polymer becomes a viscoelastic fluid when the sheath is inserted into a body at a temperature of 37 °C. In aspects where the third polymer is solid, a third polymer exhibiting a melting temperature above about 45 °C that is disposed between the third portion of the outer surface of the outer layer at at least a portion of the proximal end of the sheath and the at least a second portion of the inner surface of the outer jacket and prevent the first polymer from being leached out of the sheath into the surrounding environment.

In still further aspects, the first polymer, second polymer, and/or third polymer, while having a different melting point, can comprise the same polymer composition. It is understood that the first, second, and /or third polymer can comprise any polymer suitable for the disclosed operation conditions. In certain nonlimiting aspects, the first polymer, second polymer, and/or third polymer can comprise polyethylene glycol (PEG). In exemplary aspects where the first polymer comprises PEG, the first polymer has a molecular weight from about 500 to about 1,500 g/mol, including exemplary values of about 600, about 700, about 800, about 900, about 1,000, about 1,100, about 1,200, about 1,300, and about 1,400 g/mol. In yet other aspects the second and/or the third polymers when comprise PEG, can have a molecular weight from about 1,500 to about 3,500 g/mol, including exemplary values of about 1,600, about 1,700, about 1,800, about 1,900, about 2,000, about 2,100, about 2,200, about 2,300, about 2,400, about 2,500, about 2,600, about 2,700, about 2,800, about 2,900, about 3,000, about 3,100, about 3,200, about 3,300, about 3,400 g/mol. In yet other aspects, the second and/or third polymer can have a composition different from the first polymer. In still further aspects, the second and/or third polymer can comprise polyethylene oxide. It is understood that in such aspects, the second and the third polymer, while required to stay solid at temperatures above 45 °C, need not to be rigid and affect the flexibility of the disclosed sheath. It is further understood that the first polymer can have any thickness that can provide for the desired result. In yet other aspects, if present, the second and the third polymers can have any thickness that can ensure sealing between the outer layer and the outer jacket.

In still further aspects, the inner layer can comprise two or more folded portions. In such aspects, the first polymer can be disposed, as described above, between each of the folded portions and the outer layer.

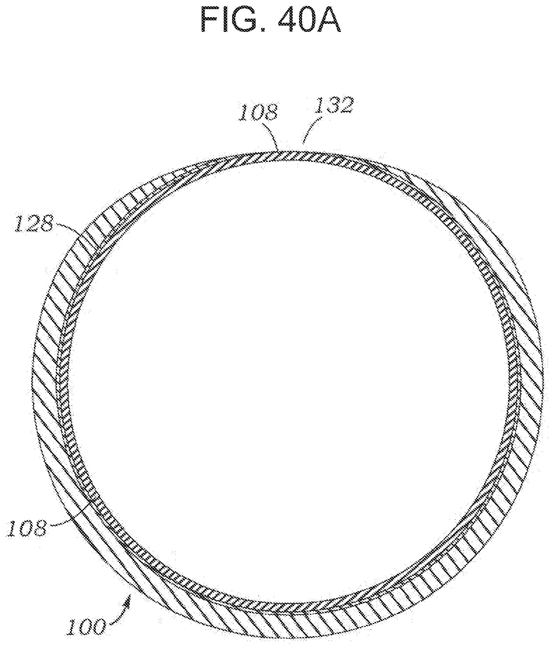

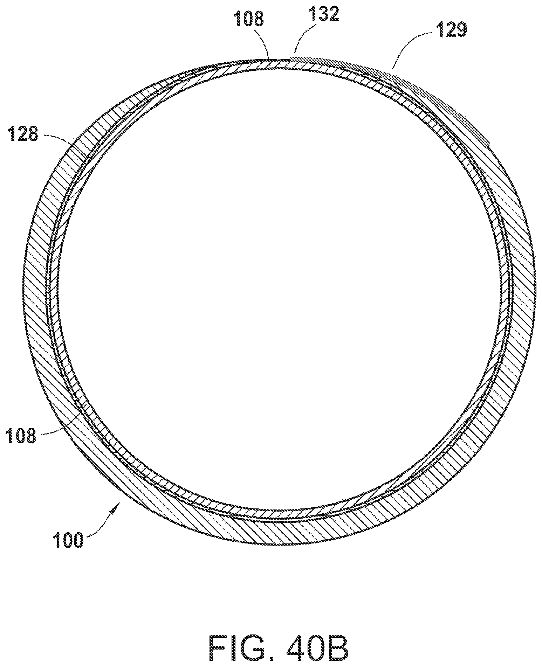

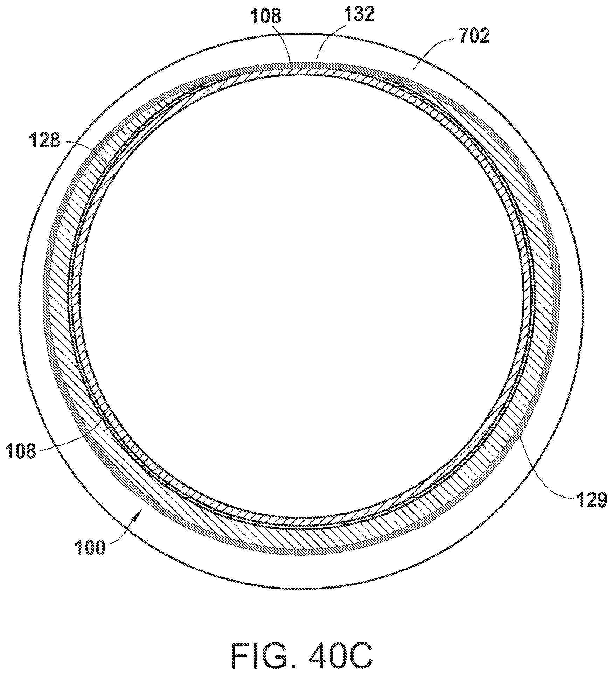

In yet further aspects, the sheath **100,** whether with or without the first polymer, is configured to expand from a resting configuration (FIG. 39A-B and 39E) to an expanded configuration shown in FIG. 40A-C. In the expanded configuration with (**FIG. 40B**) or without the presence (**FIG. 40A**) of the first polymer **129**, an annular gap **132** can form between the longitudinal edges of the overlapping portion **120** and the underlying portion **122** of the outer layer **110.** As the sheath **100** expands at a particular location, the overlapping portion **120** of the outer layer **110** can move circumferentially with respect to the underlying portion **122** as the folded portion **118** of the inner layer **108** unfolds. This movement can be facilitated by the use of a low-friction material for inner layer **108**, such as PTFE. This movement can be further facilitated by the presence of the first polymer that forms an intermediate layer that is a viscoelastic fluid at a temperature between (30 °C and 45 °C). This viscoelastic fluid behaves as a lubricant and decreases the friction between the inner layer and the outer layer. In yet other aspects, where the first polymer is present within the folding portion, the presence of the viscoelastic fluid can decrease the friction between the folded parts during the expansion. In further aspects, the improved lubrication results in the reduction of the insertion force when the medical device is inserted into the sheath. In such aspects (not shown in **FIG.40**), when the sheath is inserted into the body and is in an expanded configuration, the first polymer is present as a viscoelastic lubricating fluid. The presence of the first polymer, as disclosed above, can reduce the insertion force by at least 5 %. Further, the folded portion **118** can at least be partially separated and/or unfold to accommodate a medical device having a diameter larger than that of lumen **116** in the resting configuration. As shown in **FIG. 40**, in some aspects, the folded portion of the inner layer **108** can completely unfold so that the inner layer **108** forms a cylindrical tube at the location of the expanded configuration. It is further understood that the presence of the first polymer on the outer layer in the expanded configuration can further assist in lubricating the sheath as it moves throughout the vessels of interest.

The sheath **100** can be configured such that it locally expands at a particular location corresponding to the location of the medical device along the length of the lumen **116**, and then locally contracts once the medical device has passed that particular location. Thus, a bulge may be visible, traveling longitudinally along the length of the sheath as a medical device is introduced through the sheath, representing continuous local expansion and contraction as the device travels the length of the sheath **100.** In some aspects, each segment of the sheath **100** can locally contract after removal of any radial outward (insertion) force such that it regains the original resting diameter of lumen **116.** In such aspects, the folded portion and the first polymer return to their original position. In yet further aspects, the first polymer solidifies when the sheath is withdrawn from the body and is at a temperature below 30 °C.

In some aspects, each segment of the sheath **100** can locally contract after removal of any radial outward force such that it at least partially returns to the original resting diameter of lumen **116.**

The layers **108, 110** of sheath **100** can be configured, as shown in **FIG. 39A-****G** along at least a portion of the length of the sheath **100.** In some aspects, the layers **108, 110** can be configured as shown in FIG. 39A-G along the length A (FIG. **35**) extending from a location adjacent to the soft tip portion **102** to a location closer to the proximal end **106** of the sheath **100.** In this matter, the sheath is expandable and contractable only along a portion of the length of the sheath corresponding to length A (which typically corresponds to the section of the sheath inserted into the narrowest section of the patient's vasculature).

### METHODS

Methods of making a sheath are also disclosed. Some of the methods disclosed herein comprise providing a mandrel having a first diameter, providing a first tube having a second diameter, the second diameter being larger than the first diameter, mounting the first tube on the mandrel, gathering excess material of the first tube and folding the excess material to one side to form a folded portion of the inner layer. A second tube can then be provided, and the second tube can be cut to form a coiled layer. An adhesive can be applied to at least a portion of the coiled layer, and the coiled layer can be mounted on the first tube such that the adhesive is positioned between the first tube and the coiled layer. The folded portion can be lifted in order to position a portion of the coiled layer under the folded portion.

In yet other aspects, the methods can include applying heat to the first tube, coiled layer, and mandrel so as to thermally fuse the first tube and the coiled layer together. In some aspects, an elastic outer cover can be secured to the outer surface of the coiled layer. In yet other aspects, a soft tip portion can be coupled to a distal end of the expandable sheath to facilitate passing the expandable sheath through a patient's vasculature.

Various methods can be used to produce the sheaths discussed above and below throughout the present disclosure. For example, and without limitation, a method of making the sheath shown in **FIGS. 2A-2D** can comprise providing a mandrel and applying an inner layer on the mandrel, such as by spray coating or dip coating the mandrel. An intermediate layer, such as a mesh structure, can then be mounted on the inner layer. An outer layer can be applied over the intermediate layer, such as by a second spray coating or dip coating step. Methods can comprise etching or surface treating at least a portion of the inner layer. Also, methods can comprise providing one or more notches and/or cuts in the inner layer and/or the outer layer. Cuts and/or notches can be provided by, for example, laser cutting or etching one or more layers.

In some aspects, in the methods of making the sheaths, such as illustrated in **FIGS. 2A-2D****,** the layers can be pre-formed and mounted on a mandrel and then fused or thermally bonded together. For example, in one aspect, an inner layer is applied to a mandrel. An intermediate layer can be applied to the outer surface of the inner layer. An outer layer can be applied to the outer surface of the intermediate layer. Heat shrink tubing can be applied and the assembly heated, such that the inner layer, the intermediate layer, and/or the outer layer are thermally bonded and compressed together under the heat shrink tubing.

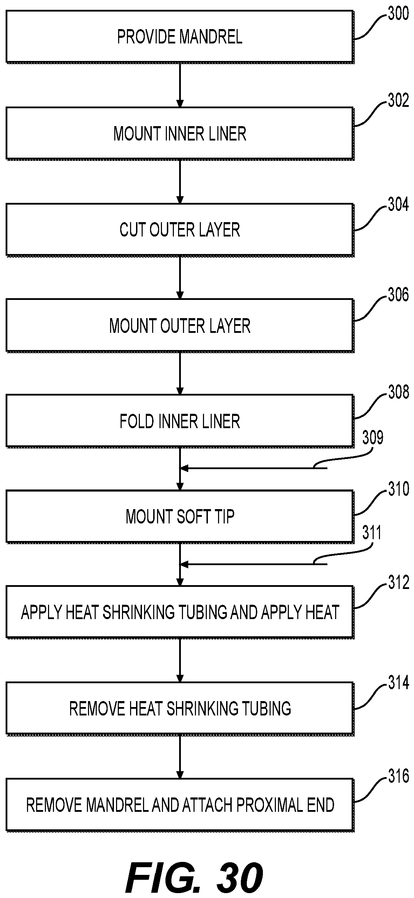

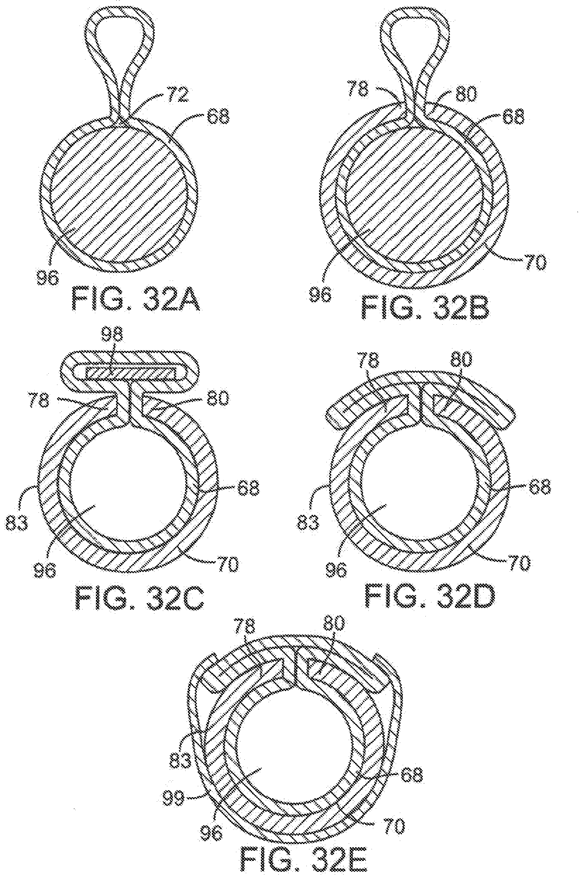

**FIG. 30** illustrates a block diagram of the method of producing a sheath, in one aspect, for use with a delivery apparatus in minimally invasive surgery. One or more mandrels can be provided (step **300**). The mandrel can be provided with an exterior coating, such as a Teflon^{®} coating, and the mandrel's diameter can be predetermined based on the desired size of the resulting sheath. A liner that will become the inner polymeric layer of the sheath can be made of any material described herein, for example, and without limitation, it can comprise a PTFE or high density polyethylene (HDPE), can be mounted on the mandrel (step **302**). The liner can be etched and/or surface treated prior to being mounted on the mandrel, according to conventional etching and surface treatment methods, as described above. **FIG. 32A** illustrates a section view of a sheath at steps **300** and **302** of **FIG. 30****.** A coated mandrel **96** is inserted within the lumen **72** of the inner polymeric layer **68.** The circumference of the inner polymeric layer **68** is larger than the circumference of the mandrel **96,** such that an excess portion of the inner polymeric layer **68** can be gathered above the mandrel **96.**

**A** layer of material that will become the outer polymeric tubular layer can be cut or notched through all, substantially all, or a part of the thickness of the layer (step **304**). It is understood that the outer layer can comprise any materials described herein without any limitations. In some unlimiting aspects, the outer layer can comprise polyurethane or polyolefin. Such a cut or notch can extend longitudinally along the length of the layer and can extend along substantially the entire length of the outer polymeric tubular layer. In alternative aspects, the cut or notch can be provided along only a portion of the outer polymeric tubular layer. For example, the outer polymeric tubular layer can be cut starting at the distal end of the outer polymeric tubular layer, with the cut ending before the proximal end of the outer polymeric tubular layer. In one aspect, the cut can end at a transition, where the outer diameter of the outer polymeric tubular layer increases or decreases. In one exemplary aspect, the cut or notch can extend longitudinally along about 50%, 55%, 60%, 70%, or 75% of the length of the sheath.

The cut or notched outer polymeric tubular layer can be applied, positioned, adhered, mounted, thermally fused or bonded, dip coated, and/or otherwise coupled to the etched inner liner (step **306**). **FIG. 32B** shows a section view of the sheath at step **306** of **FIG. 30****,** with outer polymeric tubular layer **70** applied to the inner polymeric layer **68** such that a portion of the inner polymeric layer **68** extends between the cut formed between first and second portions **78, 80** of the outer polymeric tubular layer **70.**

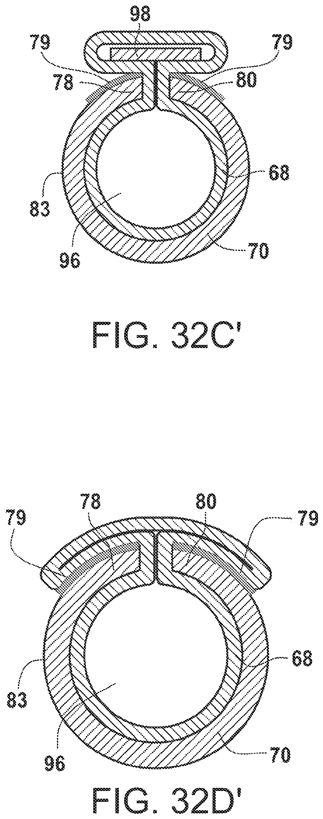

In alternative aspects, the outer polymeric tubular layer can be notched or cut after being mounted on the inner liner/mandrel assembly. The outer polymeric tubular layer can optionally be provided with a hydrophilic coating and/or provided with additional layers, such as being dip coated with polyurethane. Some portion of the inner liner can protrude through the cut in the outer polymeric tubular layer after such an outer polymeric tubular layer is mounted onto the inner liner/mandrel arrangement. Using, for example, a split tool, the protruding portion of the inner liner can be folded down onto the outer surface of the outer polymeric tubular layer (step **308**). In some aspects, the protruding portion of the inner liner is folded down along the entire length of the resulting sheath, while in other aspects, the protruding portion of the inner liner is only present along a portion of the length of the sheath or is only folded down along a portion of the length of the resulting sheath. **FIG. 32C** shows an exemplary section view of the sheath at step **308** of **FIG. 30****.** A split tool **98** is used to fold the excess portion of inner polymeric layer **68** over a portion of the outer surface **83** of the outer polymeric tubular layer **70.** **FIG. 32D** shows a section view of the sheath after completion of step **308** of **FIG. 30****.** Split tool **98** can then be removed, and folding of the excess portion of the inner polymeric layer **68** can be completed. **FIG. 32E** shows a section view of an outer covering, such as outer polymeric covering **99,** that can be applied such that it overlaps a portion of the folded portion of inner polymeric layer **68.** The outer polymeric covering **99** contacts at least a portion of the outer surface **83** of the outer polymeric tubular layer **70.** A soft, atraumatic tip can be provided at the distal end of the resulting sheath (step **310**). The first polymer is applied between at least the first portion of the outer surface of the outer layer and the inner portion of the at least one folded portion of the inner layer such that it forms an intermediate layer between the inner portion of the at least one folded portion and the at least first portion of the outer surface of the outer layer. In some aspects, the first polymer can be applied in step **309** before mounting the soft tip in step **310.** In yet other exemplary aspects, the first polymer can be applied in step **311,** after the mounting the soft tip in step **310.** In the exemplary aspects shown in **FIG. 32C'****,** the first polymer **79** can be applied between the outer surface of the outer layers **78** and/or **80** and the folded portion overlaying these layers. In such aspects, the first polymer can be applied by any known in the art methods. In some aspects, prior to the step of applying, the first polymer can be provided as a solid or as a viscoelastic fluid. In certain aspects, to ensure proper adhesion between the folded portion and the outer surface of the outer layer, the first polymer, if provided as a solid, is melted to form the viscoelastic fluid prior to the step of applying the first polymer. In still further aspects, the first polymer can be transformed into the viscoelastic fluid by any known in the art methods, for example, and without limitation by heating the polymer in an oven or any other known in the art heater (IR, UV, etc.,) above its melting point. In still further aspects, the first polymer is applied as viscoelastic fluid at a temperature above about 30 °C, above about 35 °C, above about 40 °C, or above about 45 °C. It is further understood that the temperature the polymer is exposed to can be chosen based on the specific polymer. In yet further aspects and as it could be readily understood by one of ordinary skill in the art, the upper temperature the polymer is exposed to needs to be controlled to avoid adverse effects on the polymer structure and characteristics as well as adverse effects on the sheath components when it inadvertently exposed to undesired high temperatures.

In still further aspects, the first polymer can be applied as the viscoelastic fluid by any methods known in the art. In certain aspects and without limitation, the first polymer can be applied with a brush. In yet other aspects, it can be applied as a fluid and spread over the desired area with any tool known in the art. Again, the disclosed methods for applying the viscoelastic fluids are only exemplary and non-limiting, and any methods of applying the viscoelastic fluid to any surface can be utilized.

In still further aspects, after the step of applying, the sheath is cooled to a temperature below 30 °C to ensure that the first polymer **79** is solidified and can adhere the folded portion to the outer surface of the outer layer **78** or **80** (as shown in the exemplary aspect of FIG. 32D')**.**

Additional layers can also be applied if desired. For example, the sheath can also comprise a tie layer, as described above. In such aspects, the tie layer is disposed between at least a second portion of the inner surface of the outer layer and at least a portion of the outer surface of the inner layer, such that it is configured to seal the at least a second portion of the inner surface of the outer layer with the at least a portion of the outer surface of the inner layer. Any described above tie layers can be utilized.

In yet other aspects, the methods described herein comprise positioning an outer jacket comprising an inner surface and outer surface such that it extends at least partially around the outer layer and such that the inner surface of the outer jacket overlies the outer surface of the outer layer.

In still further aspects, the methods described herein comprise the sheath where the inner surface of the outer layer comprises a third portion configured to seal with the outer surface of the inner layer at at least a portion of the distal end of the sheath. In yet other aspects, the methods described herein comprise the sheath where the inner surface of the outer layer comprises a fourth portion configured to seal with the outer surface of the inner layer at at least a portion of the proximal end of the sheath.

In still further aspects, the methods described herein can comprise the sheath where the outer surface of the outer layer comprises a second portion configured to seal with at least a first portion of the inner surface of the outer jacket at at least a portion of the distal end of the sheath. In yet other aspects, the methods described herein comprise the sheath where the outer surface of the outer layer comprises a third portion configured to seal with at least a second portion of the inner surface of the outer jacket at at least a portion of the proximal end of the sheath.

In such aspects, the methods disclosed herein can further comprise a step of applying a second polymer exhibiting a melting temperature above about 45 °C between the second portion of the outer surface of the outer layer at at least a portion of the distal end of the sheath and the at least a first portion of the inner surface of the outer jacket.

In such aspects, the methods disclosed herein can further comprise a step of applying the third polymer exhibiting a melting temperature above about 45 °C between the third portion of the outer surface of the outer layer at at least a portion of the proximal end of the sheath and the at least a second portion of the inner surface of the outer jacket.

In the aspects where the second and/or the third polymers are present, these polymers can be present in the solid or viscoelastic form prior to the application. In still further aspects, during the application step, the second and/or the third polymers are present in the viscoelastic form. In yet other aspects, the second and/or the third polymers, when present as a solid prior to the step of applying, can be transformed into the viscoelastic form by heating above their melting temperature. In still further aspects, the second and/or third polymers can be transformed to the viscoelastic fluid by any known in the art methods, for example, and without limitation by heating the polymer in an oven or any other known in the art heater (IR, UV, etc.,) above its melting point. In still further aspects, the first polymer is applied as viscoelastic fluid at a temperature above about 45 °C, or above about 50 °C, or above about 55 °C. It is further understood that the temperature the second and/or third polymers are exposed to can be chosen based on the specific polymers. In yet further aspects and as it could be readily understood by one of ordinary skill in the art, the upper temperature the second and/or third polymers are exposed to needs to be controlled to avoid adverse effects on the polymer structures and characteristics as well as adverse effects on the sheath components when it inadvertently exposed to undesired high temperatures.

In still further aspects, the second and/or third polymers can be applied as the viscoelastic fluid by any methods known in the art. In certain aspects and without limitation, the second and/or third polymer can be applied with a brush. In yet other aspects, it can be applied as a fluid and spread over the desired area with any tool known in the art. Again, the disclosed methods of application are only exemplary and non-limiting, and any methods of applying the viscoelastic fluid to any surface can be utilized. In still further aspects, the steps of applying the second polymer and the third polymer can occur substantially concurrently. In still further aspects, the steps of applying the first polymer, the second polymer, and the third polymer occur substantially concurrently. As described herein, the first, second, and third polymer can comprise the same or different composition. In still further aspects, the first, the second, and/or the third polymer can have a molecular mass in any of the disclosed above ranges. In still further aspects, it is understood that due to the difference in the melting temperature, if present, the second and/or third polymers remain solid when the sheath is introduced into a body having a temperature of 37 °C.

In still further aspects, the sheath formed according to the disclosed methods exhibits at least about a 5% reduction in insertion force when inserted into a body having a temperature of 37 °C when compared to a substantially identical reference sheath without the first polymer.

In still further aspects, a layer of heat shrink tubing, such as, for example, and without limitation, fluorinated ethylene propylene (FEP) heat shrink tubing, can be positioned over the entire assembly (step **312**). An appropriate amount of heat is applied, thus shrinking the heat shrink tubing and compressing the layers of the sheath together, such that components of the sheath can be thermally bonded or fused together where desired. Once the components of the sheath have been bonded together, the heat shrink tubing can be removed (step **314**). Finally, the proximal end of the sheath can be adhered to or otherwise attached to a housing of a catheter assembly, and the sheath can be removed from the mandrel (step **316**). It is understood that the first polymer is applied before step **312.**

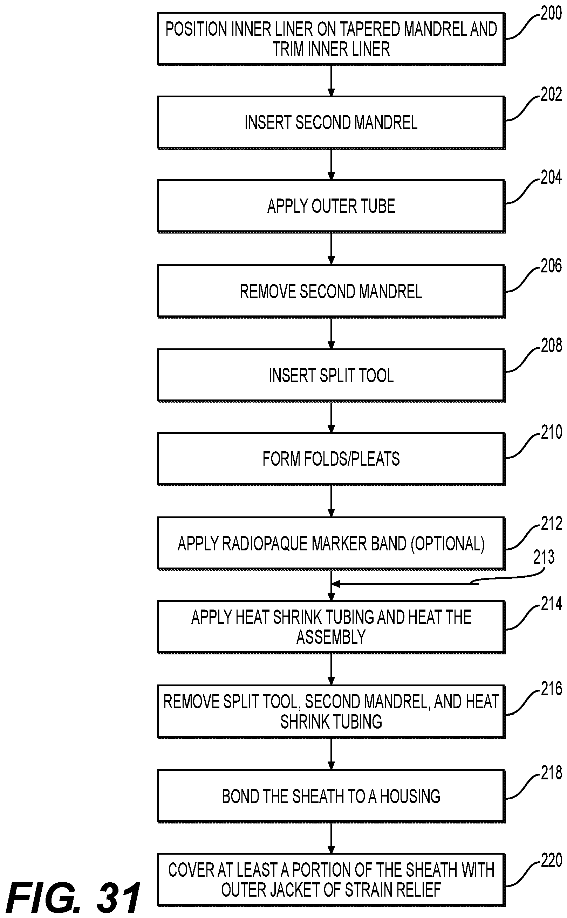

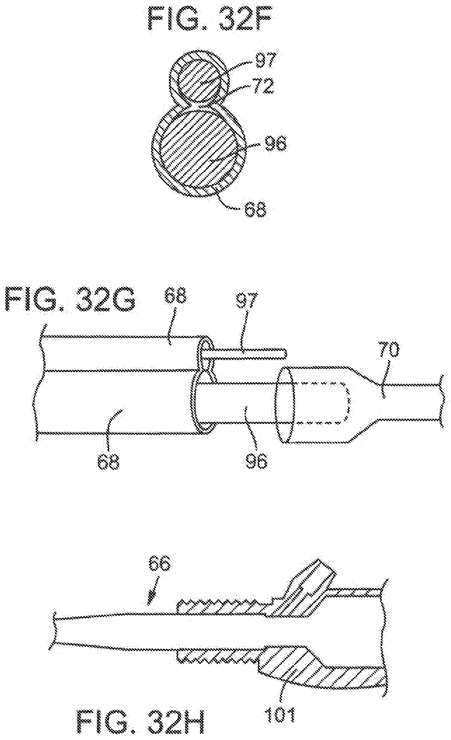

**FIG. 31** illustrates a block diagram of an alternative aspect of a method of making a sheath. An inner liner, comprising any materials described herein, for example, and without limitation, an etched or not etched PTFE or high density polyethylene (HDPE), can be applied to a tapered mandrel, such as a 16 Fr tapered mandrel, and trimmed to an appropriate length (step **200**). A second mandrel, such as a 0.070 inches diameter mandrel, can be inserted in the lumen of the inner liner such that the mandrels are arranged side by side in the inner liner (step **202**). **FIG. 32F** shows a section view of a sheath at steps **200** and **202** of **FIG. 31****.** An inner liner or inner polymeric layer **68** is applied on a first tapered mandrel **96.** A second mandrel **97** is inserted into the lumen **72** of the inner polymeric layer **68** created by the excess portion of the inner polymeric layer **68,** as described.

A notched or cut outer polymeric tubular layer, such as high density polyethylene tubing that has been notched or cut longitudinally, can be slid onto the tapered mandrel and a portion of the inner liner, starting at the distal end of the tapered mandrel (step **204**). The second mandrel can then be removed (step **206**). **FIG. 32G** illustrates a perspective view of the sheath at steps **204** and **206** of **FIG. 31****.** A polymeric outer tubular layer **70** having a longitudinal cut is applied over the tapered mandrel **96** and inner polymeric layer **68.** The outer tubular layer conforms to the portion of the inner polymeric layer around the tapered mandrel **96,** and the portion of the inner polymeric layer **68** around the second mandrel **97** extends through the longitudinal cut in the outer polymeric tubular layer **70.**

**A** split tool can be inserted into the portion of the lumen of the inner liner that was previously occupied by the second mandrel (step **208**). The split tool can then be used to form folds and/or pleats in the excess portion of the inner liner, which now extends through the longitudinal cut in the outer polymeric tubular layer (step **210**). The first polymer, as described above, is applied (step **213**), for example, between the folded portion and the outer surface of the outer portion, as shown in **FIG. 39B****.** However, the first polymer can also be applied, as shown in FIG. 39C-D. The second and the third polymers, as described above, can also be applied. A radiopaque marker band can optionally be applied at the distal end of the sheath (step **212**). It is understood that if the radiopaque marker band is present, the first polymer can be applied before or after the application of this band at the distal end of the sheath. It is further understood that the first polymer and, if present, the second and/or third polymers are applied before the heat shrink tubing is applied over the sheath in the next step. Heat shrink tubing, such as FEP heat shrink tubing, can be applied over the entire sheath, and heat can be applied to compress the components of the sheath and bond or fuse them together (step **214**). The split tool, heat shrink tubing, and second mandrel can then be removed (step **216**). The sheath can then be utilized with a delivery apparatus, such as by bonding the proximal end of the sheath to a polycarbonate housing of a delivery apparatus or catheter assembly (step 21**8**). In still further aspects, an additional step **220** can be optionally present and comprise a step of covering the whole length of the sheath or at least a portion of the sheath with an outer jacket or strain relief.

**FIG. 32H** illustrates an elevation view of the sheath at step **218** of **FIG. 31****.** The sheath **66,** made according to described methods and processes, can be attached or bonded to a housing **101,** such as by bonding the proximal end of the sheath **66** to the polycarbonate housing **101.**

In another example, disclosed expandable sheaths can be made using a reflowed mandrel process. A mandrel can be provided, with the size of the mandrel defining the inner diameter of the sheath lumen in its resting configuration. A tube of material, such as a PTFE or HDPE tube that will become the sheath's inner liner, can be provided with an inner diameter greater than that of the mandrel (e.g., a 9 mm PTFE tube can be mounted on a 6 mm mandrel). The PTFE or HDPE tube can be mounted on the mandrel and prepared into the final folded configuration by folding the excess material of the tube over to one or both sides. Another HDPE tube that will serve as the outer layer can then be placed over the PTFE or HDPE liner. The first polymer can then be disposed between the folding portion of the inner layer and the outer surface of the outer layer. Additional layers and polymers can also be added. The formed layer assembly can then be thermally fused together. For example, a reflow process can be performed where the assembly is heated to a temperature high enough such that the inner and/or outer layers are at least partially melted and are then fused together as the heat is removed and the assembly cools. It is understood that if this procedure is performed and the assembly is heated above the melting temperature of the first polymer, the second polymer, and/or third polymer is present, the polymers will melt but will re-solidify as the heat is removed, and the assembly cools to form the sheath as disclosed herein.

An elastic cover can be placed over at least part of the fused layers (e.g., over a proximal section of the sheath) and held in place using a thermal process. In some aspects, the same thermal process can bond the layers of the sheath and the elastic cover. In other aspects, a first thermal process can be used to fuse the layers of the sheath, and a second thermal process can be used to secure the elastic cover to the sheath. In some aspects, the elastic cover can be heat shrink tubing that is applied over the expandable sheath and heated to a temperature high enough to cause the tubing to shrink around the sheath. In some aspects, a distal soft tip can then be attached to the shaft of the expandable sheath.

In some aspects, the outer layer can be co-extruded with an adhesive layer or a tie layer. It is understood that any of the adhesive layers or the tie layer can comprise any materials disclosed above. For example and without limitation, the adhesive layer (tie layer) can comprise a polyurethane material such as Tecoflex, or polymer, copolymer, or terpolymer such as maleic anhydride modified polyolefin, for example, and without limitation, Orevac^{®} (commercially available from Arkema), ethylene acrylic acid copolymer, such as DOW Chemical Primacor^{®}, ethylene acrylate copolymer such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymer such as Lotader^{®} or Orevac^{®} (commercially available from Arkema). The tie layer can be disposed at at least a second portion of the inner surface of the outer layer, such that it is configured to seal the at least a second portion of the inner surface of the outer layer with at least a portion of the inner layer. In such aspects, the tie layer will be positioned between the inner and outer layers in the completed sheath. In these aspects, an exemplary outer HDPE tube can be provided with a coating of the tie layer, such as, for example, Tecoflex, on the inner surface. The outer HOPE tube can be slit along the length of the tube to open and flatten it and then cut using a template in some aspects.

For example, for specific applications, portions of the outer layer can be cut and removed using a template. The cut outer HDPE layer can then be placed on the inner layer on the mandrel. In some aspects, only a portion of the outer layer will have the adhesive layer. In these aspects, the sections without the adhesive layer (or tie layer) can only be partially fused to the inner layer. In some aspects, the entire inner surface of the outer layer can have the tie layer disposed on, and the inner surface of the outer layer can be positioned so that it contacts the inner layer on the mandrel. To position the inner and outer layers, as shown in the sheath of FIG. 39A-D, the folded portion of the inner layer can be lifted up, and an edge of the outer layer can be tucked beneath the fold. In still further aspects, these process steps can be done before applying the first polymer between the outer surface of the outer layer and the folder portion.

Sheaths of the present disclosure can be used with various methods of introducing a prosthetic device into a patient's vasculature. One such method comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some methods, the expandable sheath can be sutured to the patient's skin at the insertion site so that once the sheath is inserted the proper distance within the patient's vasculature, it does not move once the implantable device starts to travel through the sheath.

In some aspects, the disclosed expandable sheath can be used with other delivery and minimally invasive surgical components, such as an introducer and loader. In one aspect, the expandable sheath can be flushed to purge any air within the sheath, using, for example, flush port **103 (****FIG. 35****).** An introducer can be inserted into the expandable sheath, and the introducer/sheath combination can be fully inserted into vasculature over a guiding device, such as a 0.35" guidewire. In certain aspects, the seam formed by the intersection of the folded portion of the inner layer and the overlapping portion of the outer layer can be positioned such it is oriented downward (posterior). Once the sheath and introducer are fully inserted into a patient's vasculature, in some aspects, the expandable sheath can be sutured in place at the insertion site. In this manner, the expandable sheath can be substantially prevented from moving once positioned within the patient.

The introducer can then be removed, and a medical device, such as a transcatheter heart valve, can be inserted into the sheath, in some instances, using a loader. Such methods can additionally comprise placing the tissue heart valve in a crimped state on the distal end portion of an elongated delivery apparatus and inserting the elongated delivery device with the crimped valve into and through the expandable sheath. Next, the delivery apparatus can be advanced through the patient's vasculature to the treatment site, where the valve can be implanted.

Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (*e.g.,* the expanded configuration) just in the area where the medical device is currently located. Once the medical device passes a particular location of the sheath, the sheath can at least partially contract to the smaller diameter of its original configuration. The expandable sheath can thus be expanded without the use of inflatable balloons or other dilators. Once the medical device is implanted, the sheath and any sutures holding in place can be removed. In some exemplary aspects, the sheath is removed without rotating it.

### EXEMPLARY ASPECTS

In view of the described processes and compositions, hereinbelow are described certain more particularly described aspects of the disclosures.

A sheath for delivering a medical device, wherein the sheath has a proximal and a distal end and comprises: an expandable inner layer having an inner surface and an outer surface, wherein the inner surface of the expandable inner layer defines a lumen having a longitudinal axis and comprising at least one folded portion having an inner portion and outer portion; an outer layer having an inner surface and an outer surface and extending at least partially around the inner layer such that at least a first portion of the outer surface of the outer layer is positioned adjacent to the inner portion of the at least one folded portion of the inner layer, while a first portion of the inner surface of the outer layer is positioned adjacent to the outer portion of the at least one folded portion of the inner layer; a first polymer disposed between at least a portion of the inner layer and at least a portion of the outer layer, forming an intermediate layer, wherein, the first polymer exhibits a melting temperature from about 30 °C to about 45 °C; and wherein the at least one folded portion is configured to at least partially unfold during application of a radial outward force by passage of a medical device through the lumen of the inner layer.

The sheath of any examples herein, wherein the first polymer is disposed between the at least the first portion of the outer surface of the outer layer and the inner portion of the at least one folded portion of the inner layer.

The sheath of any examples herein, wherein the first polymer is disposed between at least the first portion of the inner surface of the outer layer and the outer portion of the at least one folded portion of the inner layer.

The sheath of any examples herein, wherein the sheath further comprises an outer jacket comprising an inner surface and outer surface extending at least partially around the outer layer such that the inner surface of the outer jacket overlies the outer surface of the outer layer.

The sheath of any examples herein, wherein the first polymer is disposed between at least a portion of the inner surface of the outer jacket and at least a portion of the outer surface of the outer layer.

The sheath of any examples herein, wherein the first polymer disposed between the first portion of the outer surface of the outer layer and the inner portion of the at least one folded portion of the inner layer along a longitudinal axis of the folded portion.

The sheath of any examples herein, wherein the sheath further comprises a tie layer disposed between at least a second portion of the inner surface of the outer layer and at least a portion of the outer surface of the inner layer, such that it is configured to seal the at least a second portion of the inner surface of the outer layer with the at least a portion of the outer surface of the inner layer.

The sheath of any examples herein, wherein the tie layer is disposed between the inner surface of the outer layer and the outer surface of the inner layer along a circumference of the inner layer along its longitudinal axis.

The sheath of any examples herein, wherein the expandable inner layer has a dynamic coefficient of friction of less than 0.4 against steel as measured according to ASTM D1894.

The sheath of any examples herein, wherein the expandable inner layer comprises a fluoropolymer.

The sheath of any examples herein, wherein the expandable inner layer comprises polytetrafluoroethylene (PTFE).

The sheath of any examples herein, wherein the outer surface of the expandable inner layer is etched.

The sheath of any examples herein, wherein the expandable inner layer is high density polyethylene (HDPE).

The sheath of any examples herein, wherein the inner surface of the outer layer comprises a third portion configured to seal with the outer surface of the inner layer at at least a portion of the distal end of the sheath.

The sheath of any examples herein, wherein the inner surface of the outer layer comprises a fourth portion configured to seal with the outer surface of the inner layer at at least a portion of the proximal end of the sheath.

The sheath of any examples herein, wherein the outer surface of the outer layer comprises a second portion configured to seal with at least a first portion of the inner surface of the outer jacket at at least a portion of the distal end of the sheath.

The sheath of any examples herein, wherein the outer surface of the outer layer comprises a third portion configured to seal with at least a second portion of the inner surface of the outer jacket at at least a portion of the proximal end of the sheath.

The sheath of any examples herein, wherein the first polymer is solid at a temperature below about 30 °C and adheres the first portion of the outer surface of the outer layer to the inner portion of the at least one folded portion of the inner layer.

The sheath of any examples herein, wherein the intermediate layer is a viscoelastic fluid at a temperature from 30 ° to about 45 °C and is a lubricant.

The sheath of any examples herein, wherein the sheath exhibits at least about 5% reduction in insertion force when inserted into a body having a temperature of 37 °C when compared to a substantially identical reference sheath without the first polymer.

The sheath of any examples herein, further comprising a second polymer exhibiting a melting temperature above about 45 °C that is disposed between the second portion of the outer surface of the outer layer at at least a portion of the distal end of the sheath and the at least a first portion of the inner surface of the outer jacket.

The sheath of any examples herein, further comprising a third polymer exhibiting a melting temperature above about 45 °C that is disposed between the third portion of the outer surface of the outer layer at at least a portion of the proximal end of the sheath and the at least a second portion of the inner surface of the outer jacket.

The sheath of any examples herein, wherein the first polymer, second polymer, and third polymer comprise the same composition.

The sheath of any examples herein, wherein the first polymer, second polymer, and third polymer comprise polyethylene glycol (PEG).

The sheath of any examples herein, wherein the first polymer has a molecular weight from about 500 to about 1,500 g/mol.

The sheath of any examples herein, wherein the second polymer and/or third polymer has a molecular weight from about 1,500 to about 3,500 g/mol.

The sheath of any examples herein, wherein the inner layer comprises two or more folded portions.

The sheath of any examples herein, wherein the folded portion comprises a first folded edge and a second folded edge and an overlapping portion extending circumferentially between the first and second folded edges, the overlapping portion comprising an overlap in a radial direction of at least two thicknesses of the inner layer, wherein the first folded edge is configured to move closer to the second folded edge to shorten the overlapping portion at a local axial location during application of a radial outward force by passage of the medical device and wherein shortening of the overlapping portion corresponds with a local expansion of the lumen.

The sheath of any examples herein, wherein the folded portion comprises a first folded edge and a second folded edge and an overlapping portion extending circumferentially between the first and second folded edges, the overlapping portion comprising an overlap in a radial direction of at least two thicknesses of the inner layer, wherein the first folded edge is configured to move closer to the second folded edge to shorten the overlapping portion at a local axial location during application of a radial outward force by passage of the medical device and wherein shortening of the overlapping portion corresponds with a local expansion of the lumen, the overlapping portion, wherein the outer layer includes a first longitudinally extending edge and a second longitudinally extending edge configured to separate as the sheath expands, the first longitudinal extending edge and an overlapping portion of the outer layer extending over the second longitudinal extending edge when the sheath is not expanded, wherein the first polymer is provided between the at least the first portion of the outer surface of the outer layer proximate the second longitudinally extending edge and the inner surface of the overlapping portion of the folded portion.

A method of making a sheath comprising: providing a sheath comprising: an expandable inner layer having an inner surface and an outer surface, wherein the inner surface of the expandable inner layer defines a lumen having a longitudinal axis and comprising at least one folded portion having an inner portion and outer portion; and an outer layer having an inner surface and an outer surface and extending at least partially around the inner layer such that at least a first portion of the outer surface of the outer layer is positioned adjacent to the inner portion of the at least one folded portion of the inner layer, while a first portion of the inner surface of the outer layer is positioned adjacent to the outer portion of the at least one folded portion of the inner layer; applying a first polymer between at least a portion of the inner layer and at least a portion of the outer layer, thereby forming an intermediate layer, wherein, the first polymer exhibits a melting temperature from about 30 °C to about 45 °C and wherein the at least one folded portion is configured to at least partially unfold during application of a radial outward force by passage of a medical device through the lumen of the inner layer.

The method of any examples herein, wherein the first polymer is applied between the at least the first portion of the outer surface of the outer layer and the inner portion of the at least one folded portion of the inner layer.

The method of any examples herein, particularly example 30 or 31, wherein the first polymer is applied between the at least the first portion of the inner surface of the outer layer and the outer portion of the at least one folded portion of the inner layer.

The method of any examples herein, further comprises positioning an outer jacket comprising an inner surface and outer surface such that it extends at least partially around the outer layer and such that the inner surface of the outer jacket overlies the outer surface of the outer layer.

The method of any examples herein, wherein the first polymer is applied between at least a portion of the inner surface of the outer jacket and at least a portion of the outer surface of the outer layer.

The method of any examples herein, wherein prior to the step of applying, the first polymer is provided as a solid or as a viscoelastic fluid.

The method of any examples herein, wherein the first polymer is applied as a viscoelastic fluid at a temperature above about 30 °C.

The method of any examples herein, wherein after the step of applying the sheath, is cooled to a temperature below 30 °C.

The method of any examples herein, wherein the sheath further comprises a tie layer disposed between at least a second portion of the inner surface of the outer layer and at least a portion of the outer surface of the inner layer, such that it is configured to seal the at least a second portion of the inner surface of the outer layer with the at least a portion of the outer surface of the inner layer.

The method of any examples herein, wherein the expandable inner layer has a dynamic coefficient of friction of less than 0.4 against steel as measured according to ASTM D1894.

The method of any examples herein, wherein the expandable inner layer comprises a fluoropolymer.

The method of any examples herein, wherein the expandable inner layer comprises polytetrafluoroethylene (PTFE).

The method of any examples herein, wherein the outer surface of the expandable inner layer is etched.

The method of any examples herein, wherein the expandable inner layer is high density polyethylene (HDPE).

The method of any examples herein, wherein the inner surface of the outer layer comprises a third portion configured to seal with the outer surface of the inner layer at at least a portion of the distal end of the sheath.

The method of any examples herein, wherein the inner surface of the outer layer comprises a fourth portion configured to seal with the outer surface of the inner layer at at least a portion of the proximal end of the sheath.

The method of any examples herein, wherein the outer surface of the outer layer comprises a second portion configured to seal with at least a first portion of the inner surface of the outer jacket at at least a portion of the distal end of the sheath.

The method of any examples herein, wherein the outer surface of the outer layer comprises a third portion configured to seal with at least a second portion of the inner surface of the outer jacket at at least a portion of the proximal end of the sheath.

The method of any examples herein, wherein the sheath exhibits at least about 5% reduction in insertion force when inserted into a body having a temperature of 37 °C when compared to a substantially identical reference sheath without the first polymer.

The method of any examples herein, further comprising applying a second polymer exhibiting a melting temperature above about 45 °C between the second portion of the outer surface of the outer layer at at least a portion of the distal end of the sheath and the at least a first portion of the inner surface of the outer jacket.

The method of any examples herein, further comprising applying a third polymer exhibiting a melting temperature above about 45 °C between the third portion of the outer surface of the outer layer at at least a portion of the proximal end of the sheath and the at least a second portion of the inner surface of the outer jacket.

The method of any examples herein, wherein the first polymer, second polymer, and third polymer comprise the same composition.

The method of any examples herein, wherein the first polymer, second polymer, and third polymer comprise polyethylene glycol (PEG).

The method of any examples herein, wherein the first polymer has a molecular weight from about 500 to about 1,500 g/mol.

The method of any examples herein, wherein the second polymer and/or third polymer has a molecular weight from about 1,500 to about 3,500 g/mol.

The method of any examples herein, wherein the inner layer comprises two or more folded portions.

The method of any examples herein, wherein the steps of applying the second polymer and the third polymer occur substantially concurrently.

The method of any examples herein, wherein the steps of applying the first polymer, the second polymer, and the third polymer occur substantially concurrently.

The method of any examples herein, wherein the folded portion comprises a first folded edge and a second folded edge and an overlapping portion extending circumferentially between the first and second folded edges, the overlapping portion comprising an overlap in a radial direction of at least two thicknesses of the inner layer, wherein the first folded edge is configured to move closer to the second folded edge to shorten the overlapping portion at a local axial location during application of a radial outward force by passage of the medical device and wherein shortening of the overlapping portion corresponds with a local expansion of the lumen.

The method of any examples herein, wherein the folded portion comprises a first folded edge and a second folded edge and an overlapping portion extending circumferentially between the first and second folded edges, the overlapping portion comprising an overlap in a radial direction of at least two thicknesses of the inner layer, wherein the first folded edge is configured to move closer to the second folded edge to shorten the overlapping portion at a local axial location during application of a radial outward force by passage of the medical device and wherein shortening of the overlapping portion corresponds with a local expansion of the lumen, wherein the outer layer includes a first longitudinally extending edge and a second longitudinally extending edge configured to separate as the sheath expands, the first longitudinal extending edge and an overlapping portion of the outer layer extending over the second longitudinal extending edge when the sheath is not expanded, wherein the step of applying a first polymer to the at least one folded portion comprises applying the first polymer between the at least the first portion of the outer surface of the outer layer proximate the second longitudinally extending edge and the inner surface of the overlapping portion of the folded portion.

In view of the many possible aspects to which the principles of the disclosed disclosure can be applied, it should be recognized that the illustrated aspects are only some examples of the disclosure and should not be taken as limiting. We, therefore, claim all that comes within the scope of these claims.

## Claims

1. A sheath (100) for delivering a medical device, wherein the sheath (100) has a proximal and a distal end (104) and comprises:
an expandable inner layer (108) having an inner surface and an outer surface, wherein the inner surface of the expandable inner layer (108) defines a lumen (116) having a longitudinal axis (X) and comprising at least one folded portion (118) having an inner portion and outer portion;
an outer layer (110) having an inner surface (130) and an outer surface and extending at least partially around the inner layer (108) such that at least a first portion of the outer surface of the outer layer (110) is positioned adjacent to the inner portion of the at least one folded portion (118) of the inner layer (108), while a first portion of the inner surface of the outer layer (110) is positioned adjacent to the outer portion of the at least one folded portion (118) of the inner layer (108);
a first polymer (129) disposed between at least a portion of the inner layer (108) and at least a portion of the outer layer (110), forming an intermediate layer,
wherein the first polymer (129) exhibits a melting temperature from about 30 °C to about 45 °C; and
wherein the at least one folded portion (118) is configured to at least partially unfold during application of a radial outward force by passage of a medical device through the lumen (116) of the inner layer (108).

2. The sheath (100) of claim 1, wherein the first polymer (129) is disposed between at least the first portion of the outer surface of the outer layer (110) and the inner portion of the at least one folded portion (118) of the inner layer (108);
and/or wherein the first polymer (129) is disposed between at least the first portion of the inner surface of the outer layer (110) and the outer portion of the at least one folded portion (118) of the inner layer (108).

3. The sheath (100) of any one of claims 1 or 2, wherein the sheath (100) further comprises an outer jacket (702) comprising an inner surface and outer surface extending at least partially around the outer layer (110) such that the inner surface of the outer jacket (702) overlies the outer surface of the outer layer (110);
preferably wherein the first polymer (129) is disposed between at least a portion of the inner surface of the outer jacket (702) and at least a portion of the outer surface of the outer layer (110).

4. The sheath (100) of any one of claims 2 or 3, wherein the first polymer (129) disposed between the first portion of the outer surface of the outer layer (110) and the inner portion of the at least one folded portion (118) of the inner layer (108) extends along a longitudinal axis (X) of the folded portion (118).

5. The sheath (100) of any one of claims 1 to 4, wherein the sheath (100) further comprises a tie layer (128) disposed between at least a second portion of the inner surface of the outer layer (110) and at least a portion of the outer surface of the inner layer (108), such that it is configured to seal the at least a second portion of the inner surface of the outer layer (110) with the at least a portion of the outer surface of the inner layer (108);
preferably wherein the tie layer (128) is disposed between the inner surface of the outer layer (110) and the outer surface of the inner layer (108) along a circumference of the inner layer (108) along its longitudinal axis (X).

6. The sheath (100) of any one of claims 1 to 5, wherein the expandable inner layer (108) comprises a fluoropolymer;
preferably wherein the outer surface of the expandable inner layer (108) is etched.

7. The sheath (100) of any one of claims 1 to 6, wherein the expandable inner layer (108) is high density polyethylene (HDPE).

8. The sheath (100) of any one of claims 1 to 7, wherein the inner surface of the outer layer (110) comprises a third portion configured to seal with the outer surface of the inner layer (108) at at least a portion of the distal end (104) of the sheath (100).

9. The sheath (100) of any one of claims 1 to 8, wherein the inner surface of the outer layer (110) comprises a fourth portion configured to seal with the outer surface of the inner layer (108) at at least a portion of the proximal end (106) of the sheath (100).

10. The sheath (100) of any one of claims 3 to 9, wherein the outer surface of the outer layer (110) comprises a second portion configured to seal with at least a first portion of the inner surface of the outer jacket (702) at at least a portion of the distal end (104) of the sheath (100);
and/or wherein the outer surface of the outer layer (110) comprises a third portion configured to seal with at least a second portion of the inner surface of the outer jacket (702) at at least a portion of the proximal end (106) of the sheath (100).

11. The sheath (100) of any one of claims 2 to 10, wherein the first polymer (129) is solid at a temperature below about 30 °C and adheres the first portion of the outer surface of the outer layer (110) to the inner portion of the at least one folded portion (118) of the inner layer (108).

12. The sheath (100) of any one of claims 1 to 11, wherein the intermediate layer (129) is a viscoelastic fluid at a temperature from 30 ° C to about 45 °C and is a lubricant;
and/or wherein the sheath (100) exhibits at least about 5% reduction in insertion force when inserted into a body having a temperature of 37 °C when compared to a substantially identical reference sheath (100) without the first polymer (129).

13. The sheath (100) of any one of claims 1 to 12, wherein the folded portion (118) comprises a first folded edge (131) and a second folded edge (133) and an overlapping portion extending circumferentially between the first and second folded edge (133)s, the overlapping portion comprising an overlap in a radial direction of at least two thicknesses of the inner layer (108), and
wherein the first folded edge (131) is configured to move closer to the second folded edge (133) to shorten the overlapping portion at a local axial location during application of a radial outward force by passage of the medical device and wherein shortening of the overlapping portion corresponds with a local expansion of the lumen (116).

14. The sheath (100) of any one of claims 2 to 13, wherein the folded portion (118) comprises a first folded edge (131) and a second folded edge (133) and an overlapping portion extending circumferentially between the first and second folded edge (133)s, the overlapping portion comprising an overlap in a radial direction of at least two thicknesses of the inner layer (108),
wherein the first folded edge (131) is configured to move closer to the second folded edge (133) to shorten the overlapping portion at a local axial location during application of a radial outward force by passage of the medical device and wherein shortening of the overlapping portion corresponds with a local expansion of the lumen (116), the overlapping portion,
wherein the outer layer (110) includes a first longitudinally extending edge (124) and a second longitudinally extending edge (126) configured to separate as the sheath (100) expands, the first longitudinal extending edge and an overlapping portion of the outer layer (110) extending over the second longitudinal extending edge when the sheath (100) is not expanded, and
wherein the first polymer (129) is provided between the at least the first portion of the outer surface of the outer layer (110) proximate the second longitudinally extending edge (126) and the inner surface of the overlapping portion of the folded portion (118).

15. A method of making a sheath (100) comprising:
providing a sheath (100) comprising:
an expandable inner layer (108) having an inner surface and an outer surface, wherein the inner surface of the expandable inner layer (108) defines a lumen (116) having a longitudinal axis (X) and comprising at least one folded portion (118) having an inner portion and outer portion;
an outer layer (110) having an inner surface and an outer surface and extending at least partially around the inner layer (108) such that at least a first portion of the outer surface of the outer layer (110) is positioned adjacent to the inner portion of the at least one folded portion (118) of the inner layer (108), while a first portion of the inner surface of the outer layer (110) is positioned adjacent to the outer portion of the at least one folded portion (118) of the inner layer (108);
applying a first polymer (129) between at least a portion of the inner layer (108) and at least a portion of the outer layer (110), thereby forming an intermediate layer, wherein the first polymer (129) exhibits a melting temperature from about 30 °C to about 45 °C; and
wherein the at least one folded portion (118) is configured to at least partially unfold during application of a radial outward force by passage of a medical device through the lumen (116) of the inner layer (108).

## Patentansprüche

1. Hülle (100) zur Zuführung einer medizinischen Vorrichtung, wobei die Hülle (100) ein proximales und ein distales Ende (104) aufweist und umfasst:
eine expandierbare innere Schicht (108) mit einer inneren Oberfläche und einer äu-βeren Oberfläche, wobei die innere Oberfläche der expandierbaren inneren Schicht (108) ein Lumen (116) mit einer Längsachse (X) definiert und mindestens einen gefalteten Abschnitt (118) mit einem inneren Abschnitt und einem äußeren Abschnitt umfasst;
eine äußere Schicht (110) mit einer inneren Oberfläche (130) und einer äußeren Oberfläche, und die sich mindestens teilweise um die innere Schicht (108) herum erstreckt, so dass mindestens ein erster Abschnitt der äußeren Oberfläche der äu-βeren Schicht (110) benachbart zu dem inneren Abschnitt des mindestens einen gefalteten Abschnitts (118) der inneren Schicht (108) positioniert ist, während ein erster Abschnitt der inneren Oberfläche der äußeren Schicht (110) benachbart zu dem äußeren Abschnitt des mindestens einen gefalteten Abschnitts (118) der inneren Schicht (108) positioniert ist;
ein erstes Polymer (129), das zwischen mindestens einem Abschnitt der inneren Schicht (108) und mindestens einem Abschnitt der äußeren Schicht (110) angeordnet ist und eine Zwischenschicht bildet,
wobei das erste Polymer (129) eine Schmelztemperatur von etwa 30 °C bis etwa 45 °C zeigt; und
wobei der mindestens eine gefaltete Abschnitt (118) ausgestaltet ist, um sich während der Anwendung einer radial nach außen gerichteten Kraft infolge des Durchgangs einer medizinischen Vorrichtung durch das Lumen (116) der inneren Schicht (108) hindurch mindestens teilweise zu entfalten.

2. Hülle (100) nach Anspruch 1, wobei das erste Polymer (129) zwischen mindestens dem ersten Abschnitt der äußeren Oberfläche der äußeren Schicht (110) und dem inneren Abschnitt des mindestens einen gefalteten Abschnitts (118) der inneren Schicht (108) angeordnet ist;
und/oder wobei das erste Polymer (129) zwischen mindestens dem ersten Abschnitt der inneren Oberfläche der äußeren Schicht (110) und dem äußeren Abschnitt des mindestens einen gefalteten Abschnitts (118) der inneren Schicht (108) angeordnet ist.

3. Hülle (100) nach einem der Ansprüche 1 oder 2, wobei die Hülle (100) des Weiteren einen äußeren Mantel (702) umfasst, der eine innere Oberfläche und eine äußere Oberfläche umfasst und sich mindestens teilweise um die äußere Schicht (110) herum erstreckt, so dass die innere Oberfläche des äußeren Mantels (702) über der äußeren Oberfläche der äußeren Schicht (110) liegt;
wobei vorzugsweise das erste Polymer (129) zwischen mindestens einem Abschnitt der inneren Oberfläche des äußeren Mantels (702) und mindestens einem Abschnitt der äußeren Oberfläche der äußeren Schicht (110) angeordnet ist.

4. Hülle (100) nach einem der Ansprüche 2 oder 3, wobei das erste Polymer (129), das zwischen dem ersten Abschnitt der äußeren Oberfläche der äußeren Schicht (110) und dem inneren Abschnitt des mindestens einen gefalteten Abschnitts (118) der inneren Schicht (108) angeordnet ist, sich entlang einer Längsachse (X) des gefalteten Abschnitts (118) erstreckt.

5. Hülle (100) nach einem der Ansprüche 1 bis 4, wobei die Hülle (100) des Weiteren eine Haftvermittlerschicht (128) umfasst, die zwischen mindestens einem zweiten Abschnitt der inneren Oberfläche der äußeren Schicht (110) und mindestens einem Abschnitt der äußeren Oberfläche der inneren Schicht (108) angeordnet ist, so dass sie ausgestaltet ist, um den mindestens einen zweiten Abschnitt der inneren Oberfläche der äußeren Schicht (110) mit dem mindestens einen Abschnitt der äußeren Oberfläche der inneren Schicht (108) zu dichten;
wobei vorzugsweise die Haftvermittlerschicht (128) zwischen der inneren Oberfläche der äußeren Schicht (110) und der äußeren Oberfläche der inneren Schicht (108) entlang eines Umfangs der inneren Schicht (108) entlang ihrer Längsachse (X) angeordnet ist.

6. Hülle (100) nach einem der Ansprüche 1 bis 5, wobei die expandierbare innere Schicht (108) ein Fluorpolymer umfasst;
wobei vorzugsweise die äußere Oberfläche der expandierbaren inneren Schicht (108) geätzt ist.

7. Hülle (100) nach einem der Ansprüche 1 bis 6, wobei die expandierbare innere Schicht (108) ein Polyethylen mit hoher Dichte (HDPE) umfasst.

8. Hülle (100) nach einem der Ansprüche 1 bis 7, wobei die innere Oberfläche der äu-βeren Schicht (110) einen dritten Abschnitt umfasst, der ausgestaltet ist, um mit der äußeren Oberfläche der inneren Schicht (108) an mindestens einem Abschnitt des distalen Endes (104) der Hülle (100) zu dichten.

9. Hülle (100) nach einem der Ansprüche 1 bis 8, wobei die innere Oberfläche der äu-βeren Schicht (110) einen vierten Abschnitt umfasst, der ausgestaltet ist, um mit der äußeren Oberfläche der inneren Schicht (108) an mindestens einem Abschnitt des proximalen Endes (106) der Hülle (100) zu dichten.

10. Hülle (100) nach einem der Ansprüche 3 bis 9, wobei die äußere Oberfläche der äußeren Schicht (110) einen zweiten Abschnitt umfasst, der ausgestaltet ist, um mit mindestens einem ersten Abschnitt der inneren Oberfläche des äußeren Mantels (702) an mindestens einem Abschnitt des distalen Endes (104) der Hülle (100) zu dichten;
und/oder wobei die äußere Oberfläche der äußeren Schicht (110) einen dritten Abschnitt umfasst, der ausgestaltet ist, um mit mindestens einem zweiten Abschnitt der inneren Oberfläche des äußeren Mantels (702) an mindestens einem Abschnitt des proximalen Endes (106) der Hülle (100) zu dichten.

11. Hülle (100) nach einem der Ansprüche 2 bis 10, wobei das erste Polymer (129) bei einer Temperatur unter etwa 30 °C fest ist und den ersten Abschnitt der äußeren Oberfläche der äußeren Schicht (110) an den inneren Abschnitt des mindestens einen gefalteten Abschnitts (118) der inneren Schicht (108) klebt.

12. Hülle (100) nach einem der Ansprüche 1 bis 11, wobei die Zwischenschicht (129 ) bei einer Temperatur von 30 °C bis etwa 45 °C ein viskoelastisches Fluid ist und ein Schmiermittel ist;
und/oder wobei die Hülle (100) mindestens etwa 5 % Reduktion einer Einführkraft zeigt, wenn sie in einen Körper mit einer Temperatur von 37 °C eingeführt wird, verglichen mit einer im Wesentlichen identischen Referenzhülle (100) ohne das erste Polymer (129).

13. Hülle (100) nach einem der Ansprüche 1 bis 12, wobei der gefaltete Abschnitt (118) eine erste gefaltete Kante (131) und eine zweite gefaltete Kante (133) umfasst und ein überlappender Abschnitt sich im Umfang zwischen der ersten und der zweiten gefalteten Kante (133 ) erstreckt, wobei der überlappende Abschnitt eine Überlappung in einer radialen Richtung von mindestens zwei Dicken der inneren Schicht (108) umfasst, und
wobei die erste gefaltete Kante (131) ausgestaltet ist, um sich näher an die zweite gefaltete Kante (133) zu bewegen, um den überlappenden Abschnitt an einer lokalen axialen Position während der Anwendung einer radial nach außen gerichteten Kraft infolge des Durchgang der medizinischen Vorrichtung zu verkürzen, und wobei Verkürzung des überlappenden Abschnitts einer lokalen Expansion des Lumens (116) entspricht.

14. Hülle (100) nach einem der Ansprüche 2 bis 13, wobei der gefaltete Abschnitt (118) eine erste gefaltete Kante (131) und eine zweite gefaltete Kante (133) und einen überlappenden Abschnitt umfasst, der sich im Umfang zwischen der ersten und der zweiten gefalteten Kante (133 ) erstreckt, wobei der überlappende Abschnitt eine Überlappung in einer radialen Richtung von mindestens zwei Dicken der inneren Schicht (108) umfasst,
wobei die erste gefaltete Kante (131) ausgestaltet ist, um sich näher an die zweite gefaltete Kante (133) zu bewegen, um den überlappenden Abschnitt an einer lokalen axialen Position während der Anwendung einer radial nach außen gerichteten Kraft infolge des Durchgangs der medizinischen Vorrichtung zu verkürzen, und wobei Verkürzung des überlappenden Abschnitts einer lokalen Expansion des Lumens (116) entspricht, der überlappende Abschnitt,
wobei die äußere Schicht (110) eine erste sich längs erstreckende Kante (124) und eine zweite sich längs erstreckende Kante (126) einschließt, die ausgestaltet sind, um auseinander zu gehen, wenn die Hülle (100) expandiert, wobei die erste sich längs erstreckende Kante und ein überlappender Abschnitt der äußeren Schicht (110) sich über die zweite sich längs erstreckende Kante erstrecken, wenn die Hülle (100) nicht expandiert ist, und
wobei das erste Polymer (129) zwischen mindestens dem ersten Abschnitt der äu-βeren Oberfläche der äußeren Schicht (110) nahe der zweiten sich längs erstreckenden Kante (126) und der inneren Oberfläche des überlappenden Abschnitts des gefalteten Abschnitts (118) bereitgestellt wird.

15. Verfahren zum Fertigen einer Hülle (100), umfassend:
Bereitstellen einer Hülle (100), umfassend:
eine expandierbare innere Schicht (108) mit einer inneren Oberfläche und einer äu-βeren Oberfläche, wobei die innere Oberfläche der expandierbaren inneren Schicht (108) ein Lumen (116) mit einer Längsachse (X) definiert und mindestens einen gefalteten Abschnitt (118) mit einem inneren Abschnitt und einem äußeren Abschnitt umfasst;
eine äußere Schicht (110) mit einer inneren Oberfläche und einer äußeren Oberfläche, und die sich mindestens teilweise um die innere Schicht (108) herum erstreckt, so dass mindestens ein erster Abschnitt der äußeren Oberfläche der äußeren Schicht (110) benachbart zu dem inneren Abschnitt des mindestens einen gefalteten Abschnitts (118) der inneren Schicht (108) positioniert ist, während ein erster Abschnitt der inneren Oberfläche der äußeren Schicht (110) benachbart zu dem äußeren Abschnitt des mindestens einen gefalteten Abschnitts (118) der inneren Schicht (108) positioniert ist;
Aufbringen eines ersten Polymers (129) zwischen mindestens einem Abschnitt der inneren Schicht (108) und mindestens einem Abschnitt der äußeren Schicht (110), wodurch eine Zwischenschicht gebildet wird, wobei das erste Polymer (129) eine Schmelztemperatur von etwa 30 °C bis etwa 45 °C zeigt; und
wobei der mindestens eine gefaltete Abschnitt (118) ausgestaltet ist, um sich während der Anwendung einer radial nach außen gerichteten Kraft infolge des Durchgangs einer medizinischen Vorrichtung durch das Lumen (116) der inneren Schicht (108) hindurch mindestens teilweise zu entfalten.

## Revendications

1. Gaine (100) destinée à poser un dispositif médical, la gaine (100) présentant une extrémité proximale et une extrémité distale (104) et comprenant:
une couche interne extensible (108) présentant une surface interne et une surface externe, la surface interne de la couche interne extensible (108) définissant une lumière (116) présentant un axe longitudinal (X) et comprenant au moins une partie pliée (118) présentant une partie interne et une partie externe;
une couche externe (110) présentant une surface interne (130) et une surface externe et s'étendant au moins partiellement autour de la couche interne (108) de telle sorte qu'au moins une première partie de la surface externe de la couche externe (110) est positionnée de manière adjacente à la partie interne de ladite au moins une partie pliée (118) de la couche interne (108), tandis qu'une première partie de la surface interne de la couche externe (110) est positionnée de manière adjacente à la partie externe de ladite au moins une partie pliée (118) de la couche interne (108);
un premier polymère (129) disposé entre au moins une partie de la couche interne (108) et au moins une partie de la couche externe (110), formant une couche intermédiaire,
le premier polymère (129) présentant une température de fusion d'environ 30 °C à environ 45 °C; et
ladite au moins une partie pliée (118) étant conçue pour se déplier au moins partiellement pendant l'application d'une force radiale vers l'extérieur par passage d'un dispositif médical à travers la lumière (116) de la couche interne (108).

2. Gaine (100) selon la revendication 1, le premier polymère (129) étant disposé entre au moins la première partie de la surface externe de la couche externe (110) et la partie interne de ladite au moins une partie pliée (118) de la couche interne (108);
et/ou le premier polymère (129) étant disposé entre au moins la première partie de la surface interne de la couche externe (110) et la partie externe de ladite au moins une partie pliée (118) de la couche interne (108).

3. Gaine (100) selon l'une quelconque des revendications 1 ou 2, la gaine (100) comprenant en outre une enveloppe externe (702) comprenant une surface interne et une surface externe s'étendant au moins partiellement autour de la couche externe (110) de telle sorte que la surface interne de l'enveloppe externe (702) recouvre la surface externe de la couche externe (110);
de préférence, le premier polymère (129) étant disposé entre au moins une partie de la surface interne de l'enveloppe externe (702) et au moins une partie de la surface externe de la couche externe (110).

4. Gaine (100) selon l'une quelconque des revendications 2 ou 3, le premier polymère (129) disposé entre la première partie de la surface externe de la couche externe (110) et la partie interne de ladite au moins une partie pliée (118) de la couche interne (108) s'étendant le long d'un axe longitudinal (X) de la partie pliée (118).

5. Gaine (100) selon l'une quelconque des revendications 1 à 4, la gaine (100) comprenant en outre une couche de liaison (128) disposée entre au moins une deuxième partie de la surface interne de la couche externe (110) et au moins une partie de la surface externe de la couche interne (108), de telle sorte qu'elle est conçue pour assurer l'étanchéité de ladite au moins une deuxième partie de la surface interne de la couche externe (110) avec ladite au moins une partie de la surface externe de la couche interne (108);
de préférence la couche de liaison (128) étant disposée entre la surface interne de la couche externe (110) et la surface externe de la couche interne (108) le long d'une circonférence de la couche interne (108) le long de son axe longitudinal (X).

6. Gaine (100) selon l'une quelconque des revendications 1 à 5, la couche interne extensible (108) comprenant un fluoropolymère;
de préférence la surface externe de la couche interne extensible (108) étant gravée.

7. Gaine (100) selon l'une quelconque des revendications 1 à 6, la couche interne extensible (108) étant du polyéthylène haute densité (HDPE).

8. Gaine (100) selon l'une quelconque des revendications 1 à 7, la surface interne de la couche externe (110) comprenant une troisième partie conçue pour assurer l'étanchéité avec la surface externe de la couche interne (108) au niveau d'au moins une partie de l'extrémité distale (104) de la gaine (100).

9. Gaine (100) selon l'une quelconque des revendications 1 à 8, la surface interne de la couche externe (110) comprenant une quatrième partie conçue pour assurer l'étanchéité avec la surface externe de la couche interne (108) au niveau d'au moins une partie de l'extrémité proximale (106) de la gaine (100).

10. Gaine (100) selon l'une quelconque des revendications 3 à 9, la surface externe de la couche externe (110) comprenant une deuxième partie conçue pour assurer l'étanchéité avec au moins une première partie de la surface interne de l'enveloppe externe (702) au niveau d'au moins une partie de l'extrémité distale (104) de la gaine (100);
et/ou la surface externe de la couche externe (110) comprenant une troisième partie conçue pour assurer l'étanchéité avec au moins une deuxième partie de la surface interne de l'enveloppe externe (702) au niveau d'au moins une partie de l'extrémité proximale (106) de la gaine (100).

11. Gaine (100) selon l'une quelconque des revendications 2 à 10, le premier polymère (129) étant solide à une température inférieure à environ 30 °C et faisant adhérer la première partie de la surface externe de la couche externe (110) à la partie interne de ladite au moins une partie pliée (118) de la couche interne (108).

12. Gaine (100) selon l'une quelconque des revendications 1 à 11, la couche intermédiaire (129 ) étant un fluide viscoélastique à une température de 30 °C à environ 45 °C et étant un lubrifiant;
et/ou la gaine (100) présentant une réduction d'au moins environ 5 % de la force d'insertion lorsqu'elle est insérée dans un corps présentant une température de 37 °C par rapport à une gaine de référence (100) sensiblement identique sans le premier polymère (129).

13. Gaine (100) selon l'une quelconque des revendications 1 à 12, la partie pliée (118) comprenant un premier bord plié (131) et un second bord plié (133) et une partie de chevauchement s'étendant de manière circonférentielle entre le premier et le second bord plié (133) , la partie de chevauchement comprenant un chevauchement dans une direction radiale d'au moins deux épaisseurs de la couche interne (108) et
le premier bord plié (131) étant conçu pour se rapprocher du second bord plié (133) afin de raccourcir la partie de chevauchement au niveau d'un emplacement axial local pendant l'application d'une force radiale vers l'extérieur par passage du dispositif médical et le raccourcissement de la partie de chevauchement correspondant à une dilatation locale de la lumière (116).

14. Gaine (100) selon l'une quelconque des revendications 2 à 13, la partie pliée (118) comprenant un premier bord plié (131) et un second bord plié (133) et une partie de chevauchement s'étendant de manière circonférentielle entre le premier et le second bord plié (133) , la partie de chevauchement comprenant un chevauchement dans une direction radiale d'au moins deux épaisseurs de la couche interne (108),
le premier bord plié (131) étant conçu pour se rapprocher du second bord plié (133) afin de raccourcir la partie de chevauchement au niveau d'un emplacement axial local pendant l'application d'une force radiale vers l'extérieur par passage du dispositif médical et le raccourcissement de la partie de chevauchement correspondant à une dilatation locale de la lumière (116), la partie de chevauchement,
la couche externe (110) comprenant un premier bord (124) s'étendant longitudinalement et un second bord (126) s'étendant longitudinalement conçus pour se séparer lorsque la gaine (100) se dilate, le premier bord s'étendant longitudinalement et une partie de chevauchement de la couche externe (110) s'étendant sur le second bord s'étendant longitudinalement lorsque la gaine (100) n'est pas dilatée et
le premier polymère (129) étant disposé entre au moins la première partie de la surface externe de la couche externe (110) à proximité du second bord (126) s'étendant longitudinalement et la surface interne de la partie de chevauchement de la partie pliée (118).

15. Procédé de fabrication d'une gaine (100) comprenant :
la fourniture d'une gaine (100) comprenant :
une couche interne extensible (108) présentant une surface interne et une surface externe, la surface interne de la couche interne extensible (108) définissant une lumière (116) présentant un axe longitudinal (X) et comprenant au moins une partie pliée (118) présentant une partie interne et une partie externe;
une couche externe (110) présentant une surface interne et une surface externe et s'étendant au moins partiellement autour de la couche interne (108) de telle sorte qu'au moins une première partie de la surface externe de la couche externe (110) est positionnée de manière adjacente à la partie interne de ladite au moins une partie pliée (118) de la couche interne (108), tandis qu'une première partie de la surface interne de la couche externe (110) est positionnée de manière adjacente à la partie externe de ladite au moins une partie pliée (118) de la couche interne (108);
l'application d'un premier polymère (129) entre au moins une partie de la couche interne (108) et au moins une partie de la couche externe (110), formant ainsi une couche intermédiaire, le premier polymère (129) présentant une température de fusion d'environ 30 °C à environ 45 °C; et
ladite au moins une partie pliée (118) étant conçue pour se déplier au moins partiellement pendant l'application d'une force radiale vers l'extérieur par passage d'un dispositif médical à travers la lumière (116) de la couche interne (108).
